# EUROPEAN PATENT APPLICATION

(11) **EP 3 669 874 A1**
(43) Date of publication of application: **24.06.2020**
(21) Application number: 18306783.4
(22) Date of filing: 20.12.2018
(51) Int. Cl.: A61K 31/47, A61K 31/706, A61P 35/00, A61K 31/4709, A61K 31/5377

(54) **QUINOLINE DERIVATIVES FOR USE IN THE TREATMENT OR PREVENTION OF CANCER**

(71) Applicant: ABIVAX, 75008 Paris (FR); Institut Curie, 75248 Paris Cedex 05 (FR); UNIVERSITE DE MONTPELLIER, 34090 Montpellier (FR); Centre National de la Recherche Scientifique, 75794 Paris Cedex 16 (FR)
(72) Inventor: POULETTY, Philippe, 75005 PARIS (FR); EHRLICH, Hartmut, 75016 PARIS (FR); SCHERRER, Didier, 34170 CASTELNAU LE LEZ (FR); TAZI, Jamal, 34380 CLAPIERS (FR)
(74) Representative: Nony

(57) **Abstract**

A **quinoline** derivative of formula (I') 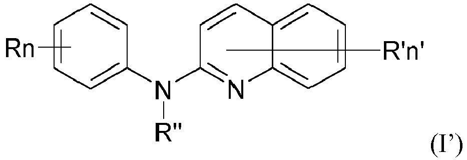 wherein R independently represent a halogen atom or a group chosen among a (C₁-C₃)fluoroalkyl group, a (C₁-C₃)fluoroalkoxy group, a -NR₁R₂ group, a (C₁-C₄)alkoxy group and a (C₁-C₃)alkyl group, said alkyl being optionally mono or di-substituted by a hydroxyl group, R' independently represent a halogen atom or a group chosen among a (C₁-C₃)alkyl group, a hydroxyl group, a -NR₁R₂ group, a morpholinyl or a morpholino group, a N-methylpiperazinyl group, a (C₁-C₃)fluoroalkyl group and a (C₁-C₄)alkoxy group, and can further be a other groups or anyone of its metabolites or pharmaceutically acceptable salt or anyone of their enantiomers or diastereoisomers, for use for treating or preventing cancer or dysplasia.

## Description

### FIELD OF THE INVENTION

The present invention has for purpose quinoline derivatives for use in the treatment of cancer.

Due to the severity of the illness, there is a permanent need to find out new drugs for preventing and/or treating cancers and/or to increase the efficacy of existing anti-cancer drugs.

There also remains a need for compounds with no or limited side-effects.

### BACKGROUND OF THE INVENTION

Some quinoline derivatives have been described in the following patent applications: WO2010/143169**,** WO2012/080953**,** WO2016/009065 and WO2016/009066 useful in the treatment of AIDS or some inflammatory diseases.

Further, some quinoline derivatives have been disclosed in WO2010/143168 useful in the treatment of some cancers.

### SUMMARY OF THE INVENTION

In the framework of the present invention, the following definitions may be given:
- effective amount: amount of a pharmaceutical compound which produces an effect on the tumour treated;
- the separate administration, simultaneous administration or administration spread out over time of a medicinal combination means that the elementary constituents of the combination, can be administered at the same time, each in one go at distinct moments, or repeatedly, or else at different moments, in particular during cycles. The elementary constituents can, in order to do this, be formulated as mixtures, only if they are administered simultaneously, or else formulated separately for the other administration schemes;
- As used herein, the term "pharmaceutically acceptable" refers to those compounds, materials, excipients, carrier, adjuvant, vehicle, compositions or dosage forms which are, within the scope of sound medical judgment, suitable for contact with the tissues of human beings and animals without excessive toxicity, irritation, allergic response or other problem complications commensurate with a reasonable benefit/risk ratio.

- The term "patient," as used herein, means an animal, preferably a mammal, and most preferably a human.
- A "combination" means a pharmaceutical composition or kit comprising a compound as defined herein after or anyone of its metabolites or pharmaceutically acceptable salts thereof, in particular 8-Chloro-N-(4-(trifluoromethoxy)phenyl)quinolin-2-amine under any form, and at least an additional anti-cancerous or anti-tumoral active agent.

Therefore, the invention relates to a **quinoline derivative** of formulas (I), (I'), (I"), (Ia), (Ib), (Ib'), (Ic), (Id), (IV), (IVa), (IVb), (IVb'), (IVc), or (IVd) as defined herein after, or a pharmaceutically acceptable salt thereof, for use for treating and/or preventing cancer or dysplasia.

Further, the present invention relates to compounds of formulas (I), (I'), (I"), (Ia), (Ib), (Ib'), (Ic), (Id), (IV), (IVa), (IVb), (IVb'), (IVc), or (IVd) as defined below, or a pharmaceutically acceptable salt thereof for use for preventing and/or inhibiting and/or treating cancer or dysplasia, or cancer metastasis or pre-tumoral lesions.

The present invention moreover relates to a method of preventing, inhibiting or treating cancer or dysplasia, which comprises at least one step consisting in administering to a patient suffering therefrom an effective amount of a compound as defined in formulas (I), (I'), (I"), (Ia), (Ib), (Ib'), (Ic), (Id), (IV), (IVa), (IVb), (IVb'), (IVc), or (IVd) below or a pharmaceutically acceptable salt thereof.

In some embodiments, a method or use of the present invention further comprises measuring a level of a compound as defined in formulas (I), (I'), (I"), (Ia), (Ib), (Ic), (Id) or (Ib') herein, or a pharmaceutically acceptable salt thereof, or a metabolite thereof, in a patient. In some embodiments, a level of a compound as defined in formulas (I), (I'), (I"), (Ia), (Ib), (Ic), (Id) or (Ib'), or a pharmaceutically acceptable salt thereof, or a metabolite thereof, is measured in a patient's biological sample. In some embodiments, a patient's biological sample is a blood, plasma, tissue, saliva and/or serum sample. In some embodiments, a method of the present invention further comprises measuring a level of a compound of formulas (IV), (IVa), (IVb), (IVb'), (IVc), or (IVd), or pharmaceutically acceptable salts thereof, in a patient. In some embodiments, a method or use of the present invention further comprises measuring a total level of compounds of formulas (I), (I') or (I") and (IV), or pharmaceutically acceptable salts thereof, in a patient. In some embodiments, a method or use of the present invention further comprises measuring a total level of compounds of formulas (Ia) and (IVa), or pharmaceutically acceptable salts thereof, in a patient. In some embodiments, a method or use of the present invention further comprises measuring a total level of compounds of formulas (Ib) and (IVb), or pharmaceutically acceptable salts thereof, in a patient. In some embodiments, a method or use of the present invention further comprises measuring a total level of compounds of formulas (Ib') and (IVb'), or pharmaceutically acceptable salts thereof, in a patient. In some embodiments, a method or use of the present invention further comprises measuring a total level of compounds of formulas (Ic) and (IVc), or pharmaceutically acceptable salts thereof, in a patient. In some embodiments, a method or use of the present invention further comprises measuring a total level of compounds of formulas (Id) and (IVd), or pharmaceutically acceptable salts thereof, in a patient.

In some embodiments, a method or use of the present invention further comprises measuring and/or monitoring a presence and/or level of a biomarker in a patient. In some embodiments, a presence and/or level of a biomarker is measured in a patient's biological sample. In some embodiments, a patient's biological sample is a blood sample. In some embodiments, a patient's biological sample is a tissue sample. In some embodiments, a biomarker measured and/or monitored in a method of the present invention is miR-124, as described in WO 2014/111892, the entire content of which is incorporated herein by reference. In some embodiments, a method or use of the present invention further comprises measuring and/or monitoring a presence and/or expression level of miR-124 in a patient prior to administering a compound or a pharmaceutically acceptable salt or composition thereof as described herein. In some embodiments, a method or use of the present invention further comprises measuring and/or monitoring a presence and/or expression level of miR-124 in a patient during the course of a treatment with a compound or a pharmaceutically acceptable salt or composition thereof as described herein. In some embodiments, a method or use of the present invention further comprises selecting a patient for a treatment with a compound or a pharmaceutically acceptable salt or composition thereof as described herein, by measuring and/or monitoring a presence and/or expression level of miR-124 in the patient. In some embodiments, a method or use of the present invention further comprises excluding a patient from a treatment with a compound or a pharmaceutically acceptable salt or composition thereof as described herein, by measuring and/or monitoring a presence and/or expression level of miR-124 in the patient. In some embodiments, a method or use of the present invention further comprises adjusting (such as increasing or decreasing) dosage regimen (such as dose amount and/or dose schedule) of a compound or a pharmaceutically acceptable salt or composition thereof as described herein to be administerd to a patient, by measuring and/or monitoring a presence and/or expression level of miR-124 in the patient.

In some embodiments, a method or use of the present invention comprises comparing a measured expression level of miR-124 in a patient to a control reference value. A control reference value to be used for comparing a measured expression level of miR-124 in a patient is obtained from a control sample. A control sample can be taken from various sources. In some embodiments, a control sample is taken from a patient prior to treatment or prior to the presence of a disease (such as an archival blood sample or tissue sample). In some embodiments, a control sample is taken from a set of normal, non-diseased members of a population. In some embodiments, a control sample is taken from a patient prior to treatment with a compound or a pharmaceutically acceptable salt or composition thereof as described herein. In some embodiments, a cell assay can be performed on a biological sample.

In some embodiments, a modulated presence and/or expression level of miR-124 in a patient compared to a control reference value indicates a cancer. In some embodiments, a modulated presence and/or expression level of miR-124 in a patient compared to a control reference value indicates an efficacy of a treatment with a compound or a pharmaceutically acceptable salt or composition thereof as described herein, which is administered to the patient. The terms "modulation" or "modulated presence and/or expression level" means the presence or expression level of a biomarker is either induced or increased, or alternatively is suppressed or decreased.

In some embodiments, a measured reduced or suppressed presence, or a decreased expression level, of miR-124 relative to a control reference value indicates a cancer. In some embodiments, a measured induced or increased presence, or an increased expression level, of miR-124 relative to a control reference value indicates an efficacy of a compound or a pharmaceutically acceptable salt or composition thereof as described herein. In some embodiments, a measured expression level of miR-124 in a patient treated with a compound or a pharmaceutically acceptable salt or composition thereof as described herein is a two-fold, four-fold, six-fold, eight-fold, or ten-fold increase relative to a control reference value.

Herein is also provided an *in vitro* or *ex vivo* use of miR-124, as a biomarker of an activity of a quinoline derivative of formula (I), (I') or (I") or anyone of its or metabolites or pharmaceutically acceptable salt thereof as defined above, on a cancer.

According to one embodiment, use and methods according to the invention may, in particular, allow for the determining of a cancer in a patient, and in particular for the follow-up of such cancer.

The use according to the present invention may be aimed at assessing a responsiveness of a patient to a treatment of cancer with the quinoline derivative according to the present invention. It may be further aimed at assessing an effectiveness of a treatment of a cancer with said quinoline derivative or at assessing a therapeutic efficacy of said quinoline derivative as a therapeutic agent for preventing and/or treating a cancer.

It may be moreover aimed at assessing a patient compliance with antitumoral treatment with said quinoline derivative.

In some embodiments, the present invention provides a use of a compound of formulas (I), (I'), (I"), (Ia), (Ib), (Ib'), (Ic), (Id), (IV), (IVa), (IVb), (IVb'), (IVc), or (IVd), or a pharmaceutically acceptable salt thereof, wherein a presence and/or expression level of miR-124 in a blood and/or tissue sample is measured to guide, dose or monitor response to the treatment.

In some embodiments, the present invention provides a use of a compound of formulas (I), (I'), (I"), (Ia), (Ib), (Ib'), (Ic), (Id), (IV), (IVa), (IVb), (IVb'), (IVc), or (IVd), or a pharmaceutically acceptable salt thereof, wherein miR-124 methylation is measured to guide therapy.

In some embodiments, the present invention provides an algorithm that combines miR-124 level and the level of a cytokine or another biomarker, or levels of compounds of formulas (I) and/or (IV) as defined herein after or pharmaceutically acceptable salts thereof, to monitor severity of a cancer, and/or to monitor efficacy a treatment, including but not limited to a treatment as described herein. In some embodiments, a treatment method as described herein comprises using an algorithm that combines miR-124 level and the level of a cytokine or another biomarker, or levels of compounds of formulas I and/or IV or pharmaceutically acceptable salts thereof, to monitor severity of a cancer, and/or to monitor efficacy the treatment.

Herein is further provided a quinoline derivative of formula (I') or anyone of its metabolites or pharmaceutically acceptable salt or anyone of their enantiomers or diastereoisomers for use according to the present invention, wherein an algorithm that combines miR-124 level and the level of cytokine or another biomarker, or levels of a compounds of formula (I') as defined herein after or anyone of its metabolites or pharmaceutically acceptable salt or anyone of their enantiomers or diasteroisomers is used to monitor severity of a cancer, and/or to monitor efficacy a treatment.

Herein is further provided an algorithm that combines miR-124 level and the level of a cytokine or another biomarker, or levels of a compound of formula (I') as defined herein after or anyone of its metabolites or pharmaceutically acceptable salt or anyone of their enantiomers or diastereoisomers to monitor efficacy a treatment.

Herein is also provided a quinoline derivative of formula (I), (I') or (I") or anyone of its or metabolites or pharmaceutically acceptable salt thereof for use as an antitumor agent intended for patients whose presence and/or expression level of miR-124 is monitored in a blood and/or tissue sample of said patient, prior to and/or during the course of said use of the antitumor agent or treatment.

Herein is also provided a quinoline derivative of formula (I), (I') or (I") or anyone of its or metabolites or pharmaceutically acceptable salt thereof for use as an antitumor agent for patients for which the presence and/or expression level of miR-124 has been measured in a blood and/or tissue sample of said patient, before, during and/or after the administration.

Herein is therefore provided a method of assessing an activity of a quinoline derivative of formula (I), (I') or (I") for preventing and/or treating cancer in a patient treated with said quinoline derivative, comprising at least the steps of:
a- measuring a presence or an expression level of at least one miRNA, said at least one miRNA being miR-124, in a first biological sample previously obtained from said patient before administering said quinoline derivative and in a second biological sample previously obtained from said patient after administering said quinoline derivative; and
b- determining if said presence or expression level is modulated in the second biological sample obtained after the treatment as compared to the second biological sample obtained before the treatment;
wherein a modulated presence or level of expression of said miRNA is indicative of an anticancerous activity of said quinoline derivative.

In some embodiments, a method or use of the present invention is used in combination with another active ingredient, in particular another anti-tumoral agent as described herein.

In some embodiments, a method or use of the present invention is used in combination with another therapy, as described herein. In some embodiments, another therapy is selected from chemotherapy, immunotherapy, radiotherapy, surgery, ultrasounds, monoclonal antibodies, and cancer vaccines.

According to the present invention, the term "preventing" or "prevention" means to reduce the risk of onset or slow the occurrence of a given phenomenon, for example, a cancer or a cancer metastasis, or dysplasia.

### DETAILED DESCRIPTION OF THE INVENTION

### Compounds

Compounds described herein may be prepared according to the synthetic routes as described in WO2010/143169, WO2012/080953, WO2016/009065 and WO2016/009066, the contents of which are incorporated herein by reference in their entireties.

In one aspect, a compound of the invention is a compound of Formula (I): 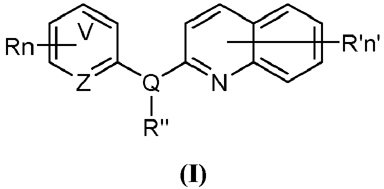 or a pharmaceutically acceptable salt thereof, wherein:
Z is C or N;
V is C or N; 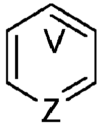 means an aromatic ring wherein V is C or N and when V is N, V is ortho, meta or para relative to Z;
each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R₁, -NR₁-C(=O)-R₁, -NR₁-C(=O)-NR₁R₂, -SO₂-NR₁R₂, -SO₃H, -O-SO₂-OR₃, -O-P(=O)-(OR₃)(OR₄), -O-CH₂-COOR₃, (C₁-C₃)alkyl, said alkyl being optionally mono- or di-substituted by a hydroxyl group, a group of formula (IIa): 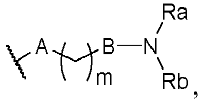 or a group of formula (IIIa): 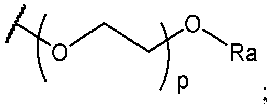
Q is N or O, provided that R" does not exist when Q is O;
each of R₁ and R₂ is independently hydrogen or (C₁-C₃)alkyl;
each of R₃ and R₄ is independently hydrogen, Li⁺, Na⁺, K⁺, N⁺(Ra)₄, or benzyl;
n is 1, 2 or 3;
n' is 1, 2 or 3;
each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy, -OP(=O)-(OR₃)(OR₄), -CN, a group of formula (IIa): 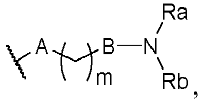 or a group of formula (IIIa): 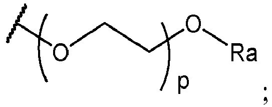
A is a covalent bond, oxygen, or NH;
B is a covalent bond or NH;
m is 1, 2, 3, 4 or 5;
p is 1, 2 or 3;
each of Ra and Rb is independently hydrogen, (C₁-C₅)alkyl, or (C₃-C₆)cycloalkyl, or
Ra and Rb form together with the nitrogen atom to which they are attached a saturated 5- or 6-membered heterocycle, said heterocycle being optionally substituted by one or more Ra, provided that when R' is a group (IIa) or (IIIa), n' may be 2 or 3 only if other R' groups are different from said group (IIa) or (IIIa); and
R" is hydrogen, (C₁-C₄)alkyl, or a group of formula (IIa) as defined above.

As defined generally above, Z is C or N.

In some embodiments, Z is C. In some embodiments, Z is N.

In some embodiments, Z is selected from those depicted in **Tables 1-3,** below.

As defined generally above, V is C or N.

In some embodiments, V is C. In some embodiments, V is N.

In some embodiments, V is selected from those depicted in **Tables 1-3,** below.

As defined generally above, 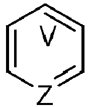 means an aromatic ring wherein V is C or N and when V is N, V is ortho, meta or para relative to Z.

In some embodiments, 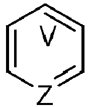 means an aromatic ring wherein V is C.

In some embodiments, 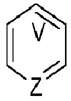 means an aromatic ring wherein V is N, and V is ortho, meta or para relative to Z. In some embodiments, V is N, and V is ortho relative to Z. In some embodiments, V is N, and V is meta relative to Z. In some embodiments, V is N, and V is para relatieve to Z.

In some embodiments, 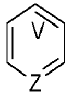 is phenyl. In some embodiments, 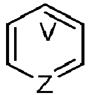 is pyridine.

In some embodiments, 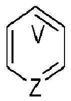 is pyridazine. In some embodiments, 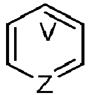 is pyrimidine.

In some embodiments, 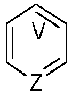 is pyrazine.

In some embodiments, 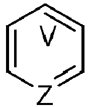 is selected from those depicted in **Tables 1-3,** below.

As described generally above, each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R₁, -NR₁-C(=O)-R₁, -NR₁-C(=O)-NR₁R₂, -SO₂-NR₁R₂, -SO₃H, -O-SO₂-OR₃, -O-P(=O)-(OR₃)(OR₄), -O-CH₂-COOR₃, or (C₁-C₃)alkyl, said alkyl being optionally mono- or di-substituted by a hydroxyl group.

In some embodiments, R is hydrogen. In some embodiments, R is halogen. In some embodiments, R is -CN. In some embodiments, R is hydroxyl. In some embodiments, R is (C₁-C₃)fluoroalkyl, said alkyl being optionally mono- or di- substituted by hydroxyl. In some embodiments, R is (C₁-C₃)fluoroalkoxy. In some embodiments, R is (C₃-C₆)cycloalkyl. In some embodiments, R is -NO₂. In some embodiments, R is -NR₁R₂. In some embodiments, R is (C₁-C₄)alkoxy. In some embodiments, R is phenoxy. In some embodiments, R is -NR₁-SO₂-NR₁R₂. In some embodiments, R is -NR₁-SO₂-R₁. In some embodiments, R is -NR₁-C(=O)-R₁. In some embodiments, R is -NR₁-C(=O)-NR₁R₂. In some embodiments, R is - SO₂-NR₁R₂. In some embodiments, R is -SO₃H. In some embodiments, R is -O-SO₂-OR₃. In some embodiments, R is -O-P(=O)-(OR₃)(OR₄). In some embodiments, R is -O-CH₂-COOR₃. In some embodiments, R is (C₁-C₃)alkyl, said alkyl being optionally mono- or di-substituted by hydroxyl.

In some embodiments, each R is independently halogen, (C₁-C₃)fluoroalkyl, (C₁-C₃)fluoroalkoxy, -NR₁R₂, (C₁-C₄)alkoxy, or (C₁-C₃)alkyl.

In some embodiments, each R is independently hydrogen, methyl, methoxy, trifluoromethyl, trifluoromethoxy, amino, halogen, or -O-P(=O)-(OR₃)(OR₄). In some embodiments, R is methyl. In some embodiments, R is methoxy. In some embodiments, R is trifluoromethyl. In some embodiments, R is trifluoromethoxy. In some embodiments, R is amino. In some embodiments, R is -O-P(=O)-(OR₃)(OR₄).

In some embodiments, each R is independently methyl, methoxy, trifluoromethyl, halogen, trifluoromethoxy, or amino.

In some embodiments, R is selected from those depicted in **Tables 1-3,** below.

As described generally above, Q is N or O, provided that R" does not exist when Q is O.

In some embodiments, Q is N. In some embodiments, Q is O, and R" does not exist.

In some embodiments, Q is selected from those depicted in **Tables 1-3,** below.

As described generally above, each of R₁ and R₂ is independently hydrogen or (C₁-C₃)alkyl.

In some embodiments, R₁ is hydrogen. In some embodiments, R₁ is (C₁-C₃)alkyl. In some embodiments, R₂ is hydrogen. In some embodiments, R₂ is (C₁-C₃)alkyl.

In some embodiments, each of R₁ and R₂ is independently selected from those depicted in **Tables 1-3,** below.

As described generally above, each of R₃ and R₄ is independently hydrogen, Li⁺, Na⁺, K⁺, N⁺(Ra)₄ or benzyl.

In some embodiments, R₃ is hydrogen. In some embodiments, R₃ is Li⁺. In some embodiments, R₃ is Na⁺. In some embodiments, R₃ is K⁺. In some embodiments, R₃ is N⁺(Ra)₄. In some embodiments, R₃ is benzyl.

In some embodiments, R₄ is hydrogen. In some embodiments, R₄ is Li⁺. In some embodiments, R₄ is Na⁺. In some embodiments, R₄ is K⁺. In some embodiments, R₄ is N⁺(Ra)₄. In some embodiments, R₄ is benzyl.

In some embodiments, each of R₃ and R₄ is independently selected from those depicted in **Tables 1-3,** below.

As described generally above, n is 1, 2 or 3.

In some embodiments, n is 1 or 2. In some embodiments, n is 1. In some embodiments, n is 2. In some embodiments, n is 3.

In some embodiments, n is selected from those depicted in **Tables 1-3,** below.

As described generally above, n' is 1, 2 or 3.

In some embodiments, n' is 1 or 2. In some embodiments, n' is 1. In some embodiments, n' is 2. In some embodiments, n' is 3.

In some embodiments, n' is selected from those depicted in **Tables 1-3,** below.

As described generally above, each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy, -O-P(=O)-(OR₃)(OR₄), -CN, a group of formula (IIa): 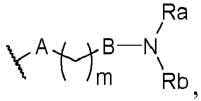 or a group of formula (IIIa):

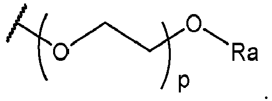

In some embodiments, R' is hydrogen. In some embodiments, R' is (C₁-C₃)alkyl. In some embodiments, R' is Hydroxyl. In some embodiments, R' is halogen. In some embodiments, R' is -NO₂. In some embodiments, R' is -NR₁R₂. In some embodiments, R' is morpholinyl. In some embodiments, R' is morpholino. In some embodiments, R' is N-methylpiperazinyl. In some embodiments, R' is (C₁-C₃)fluoroalkyl. In some embodiments, R' is (C₁-C₄)alkoxy. In some embodiments, R' is -O-P(=O)-(OR₃)(OR₄). In some embodiments, R' is -CN. In some embodiments, R' is a group of formula (IIa): 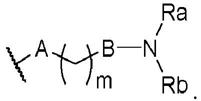 In some embodiments, R' is a group of formula (IIIa):

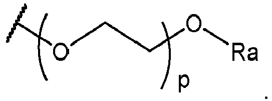

In some embodiments, R' is amino. In some embodiments, R' is methyl. In some embodiments, R' is a group of formula 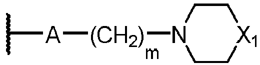 wherein A is O or NH, m is 2 or 3 and X₁ is O, CH₂ or N-CH₃, provided that when R' is such a group, n' is 1 or 2, and when n' is 2, the other R' group is different from said group.

In some embodiments, R' is a group of formula 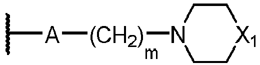 wherein A is O or NH, m is 2 and X₁ is O, CH₂ or N-CH₃, provided that when R' is such a group, n' is 1 or 2, and when n' is 2, the other R' group is different from said group.

In some embodiments, R' is a group of formula 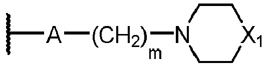 wherein A is O or NH, m is 3 and X₁ is O, CH₂ or N-CH₃, provided that when R' is such a group, n' is 1 or 2, and when n' is 2, the other R' group is different from said group.

In some embodiments, each R' is independently hydrogen, halogen, amino, methyl, -O-P(=O)-(OR₃)(OR₄), or a group of formula 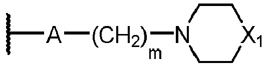 wherein A is O or NH, m is 2 or 3 and X₁ is O, CH₂ or N-CH₃, provided that when R' is such a group, n' is 1 or 2, and when n' is 2, the other R' group is different from said group.

In some embodiments, each R' is independently hydrogen, halogen, methyl, or a group of formula 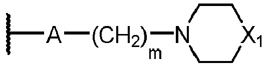 wherein A is O or NH, m is 2 and X₁ is O, CH₂ or N-CH₃, provided that when R' is such a group, n' is 1 or 2, and when n' is 2, the other R' group is different from said group.

In some embodiments, each R' is independently halogen, (C₁-C₃)alkyl, hydroxyl, -NR₁R₂, morpholinyl, morpholino, N-methylpiperazinyl, (C₁-C₃)fluoroalkyl, (C₁-C₄)alkoxy, or a group of formulas (IIa) or (IIIa) as described herein.

In some embodiments, R' is halogen or methyl.

In some embodiments, each R' is independently selected from those depicted in **Tables 1-3,** below.

As described generally above, A is a covalent bond, oxygen, or NH.

In some embodiments, A is a covalent bond. In some embodiments, A is oxygen. In some embodiments, A is NH.

In some embodiments, A is selected from those depicted in **Tables 1-3,** below.

As described generally above, B is a covalent bond or NH.

In some embodiments, B is a covalent bond. In some embodiments, B is NH.

In some embodiments, B is selected from those depicted in **Tables 1-3,** below.

As described generally above, m is 1, 2, 3, 4 or 5.

In some embodiments, m is 1. In some embodiments, m is 2. In some embodiments, m is 3. In some embodiments, m is 4. In some embodiments, m is 5.

In some embodiments, m is selected from those depicted in **Tables 1-3,** below.

As described generally above, p is 1, 2 or 3.

In some embodiments, p is 1. In some embodiments, p is 2. In some embodiments, p is 3. In some embodiments, p is 4. In some embodiments, p is 5.

In some embodiments, p is selected from those depicted in **Tables 1-3,** below.

As described generally above, each of Ra and Rb is independently hydrogen, (C₁-Cs)alkyl, or (C₃-C₆)cycloalkyl, or Ra and Rb form together with the nitrogen atom to which they are attached a saturated 5- or 6-membered heterocycle, said heterocycle being optionally substituted by one or more Ra, provided that when R' is a group (IIa) or (IIIa), n' may be 2 or 3 only if other R' groups are different from said group (IIa) or (IIIa).

In some embodiments, Ra is hydrogen. In some embodiments, Ra is (C₁-C₅)alkyl. In some embodiments, Ra is (C₃-C₆)cycloalkyl. In some embodiments, Rb is hydrogen. In some embodiments, Rb is (C₁-C₅)alkyl. In some embodiments, Rb is (C₃-C₆)cycloalkyl.

In some embodiments, Ra and Rb form together with the nitrogen atom to which they are attached a saturated 5- or 6- membered heterocycle, said heterocycle being optionally substituted by one or more Ra, provided that when R' is a group of formulas (IIa) or (IIIa), n' may be 2 or 3 only if other R' groups are different from said group of formulas (IIa) or (IIIa). In some embodiments, a saturated 5- or 6- membered heterocycle formed by Ra and Rb together with the nitrogen atom to which they are attached, as described above, optionally has an additional heteroatom selected from N, O and S.

In some embodiments, Ra and Rb form together with the nitrogen atom to which they are attached a saturated 5- or 6- membered heterocycle having an additional heteroatom selected from N, O and S, said heterocycle being substituted by one or more Ra, provided that when R' is a group of formulas (IIa) or (IIIa), n' may be 2 or 3 only if other R' groups are different from said group of formulas (IIa) or (IIIa).

In some embodiments, Ra and Rb form together with the nitrogen atom to which they are attached a saturated 5-membered heterocycle, provided that when R' is a group of formulas (IIa) or (IIIa), n' may be 2 or 3 only if other R' groups are different from said group of formulas (IIa) or (IIIa).

In some embodiments, Ra and Rb form together with the nitrogen atom to which they are attached a saturated 6-membered heterocycle, provided that when R' is a group of formulas (IIa) or (IIIa), n' may be 2 or 3 only if other R' groups are different from said group of formulas (IIa) or (IIIa).

In some embodiments, Ra and Rb form together with the nitrogen atom to which they are attached a saturated 5- or 6-membered heterocycle optionally having an additional heteroatom selected from N, O and S, said heterocycle being optionally substituted by one or more Ra, provided that when R' is a group (IIa) or (IIIa), n' may be 2 only if the other R' group is different from said group (IIa) or (IIIa).

In some embodiments, each of Ra and Rb is independently selected from those depicted in **Tables 1-3,** below.

As described generally above, R" is hydrogen, (C₁-C₄)alkyl, or a group of formula (IIa) as defined above.

In some embodiments, R" is hydrogen or (C₁-C₄)alkyl. In some embodiments, R" is hydrogen. In some embodiments, R" is (C₁-C₄)alkyl. In some embodiments, R" is a group of formula (IIa) as described herein.

In some embodiments, R" is a group of formula 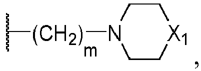 wherein m is 2 or 3, and X₁ is O, CH₂, or N-CH₃.

In some embodiments, R" is selected from those depicted in **Tables 1-3,** below.

In some embodiments, n is 1; n' is 1 or 2; R" is H; R is selected from methyl, methoxy, trifluoromethyl, halogen, trifluoromethoxy, and amino; and each R' is independently halogen, methyl, or a group 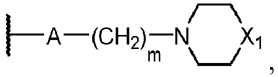 wherein A is O or NH, m is 2 or 3 and X₁ is O, CH₂ or N-CH₃, provided that when n' is 2, the other R' group is different from said group.

In some embodiments, n is 1; n' is 1; R" is H; R is selected from methyl, methoxy, trifluoromethyl, halogen, and trifluoromethoxy; and R' is halogen or methyl.

In some embodiments, a compound of the invention is a compound of formula (**Ia**): 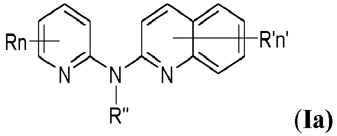 or a pharmaceutically acceptable salt thereof, wherein each of variables R, R', R", n, and n' is independently as defined above and described in embodiments herein, both singly and in combination.

In some embodiments, a compound of the invention is a compound of formula (**Ib**): 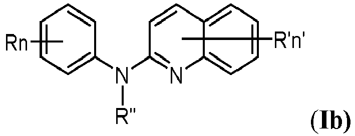 or a pharmaceutically acceptable salt thereof, wherein each of variables R, R', R", n, and n' is independently as defined above and described in embodiments herein, both singly and in combination.

In some embodiments, a compound of the invention is a compound of formula **(Ic):** 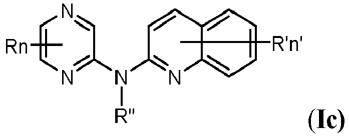 or a pharmaceutically acceptable salt thereof, wherein each of variables R, R', R", n, and n' is independently as defined above and described in embodiments herein, both singly and in combination.

In some embodiments, a compound of the invention is a compound of formula (**Ib**'): 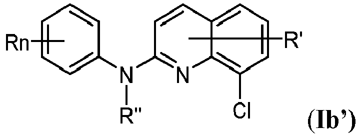 or a pharmaceutically acceptable salt thereof, wherein each of variables R, R', R", and n is independently as defined above and described in embodiments herein, both singly and in combination.

In some embodiments, a compound of the invention is a compound of formula (**Ib**), or a pharmaceutically acceptable salt thereof, wherein:
each R is independently halogen, (C₁-C₃)fluoroalkyl, (C₁-C₃)fluoroalkoxy, -NR₁R₂, (C₁-C₄)alkoxy, or (C₁-C₃)alkyl, said alkyl being optionally mono- or di-substituted by a hydroxyl group;
n is 1 or 2;
n' is 1 or 2;
each of R₁ and R₂ is independently hydrogen or (C₁-C₃)alkyl;
each of R' is independently halogen, (C₁-C₃)alkyl, hydroxyl, -NR₁R₂, morpholinyl, morpholino, N-methylpiperazinyl, (C₁-C₃)fluoroalkyl, (C₁-C₄)alkoxy, or a group of formulas (IIa) or (IIIa) as described herein;
A is a covalent bond, oxygen, or NH;
B is a covalent bond or NH;
m is 1, 2, 3, 4 or 5;
p is 1, 2 or 3;
each of Ra and Rb is independently hydrogen, (C₁-C₅)alkyl, or (C₃-C₆)cycloalkyl, or
Ra and Rb form together with the nitrogen atom to which they are attached a saturated 5- or 6-membered heterocycle optionally having an additional heteroatom selected from N, O and S, said heterocycle being optionally substituted by one or more Ra, provided that when R' is a group (IIa) or (IIIa), n' may be 2 only if the other R' group is different from said group (IIa) or (IIIa); and
R" is hydrogen or (C₁-C₄)alkyl.

In some embodiments, a compound of the invention is a compound of formula (**Ib**), or a pharmaceutically acceptable salt thereof, wherein each R' is independently hydrogen, halogen, (C₁-C₃)alkyl, or a (C₁-C₄)alkoxy group, said alkyl being optionally mono- or di-substituted by a hydroxyl group; R" is hydrogen or (C₁-C₄)alkyl; n is 1 or 2; n' is 1 or 2; when n is 1, R is (C₁-C₃) fluoroalkoxy, NR₁R₂, or phenoxy, wherein each of R₁ and R₂ is independently (C₁-C₃)alkyl; and when n is 2, one of the two R groups is (C₁-C₃) fluoroalkoxy and the other R group is (C₁-C₃)alkyl.

In some embodiments, a compound of the invention is a compound of formula (**Ib**), or a pharmaceutically acceptable salt thereof, wherein each R is independently (C₁-C₃)fluoroalkoxy; each R' is independently hydrogen, halogen, (C₁-C₃)alkyl, or (C₁-C₄)alkoxy; R" is hydrogen or (C₁-C₄)alkyl; n is 1; and n' is 1 or 2.

In some embodiments, a compound of the invention is a compound of formula (**Ib**'), or a pharmaceutically acceptable salt thereof, wherein each R is independently hydrogen, halogen, (C₁-C₃)alkyl, -NR₁R₂, (C₁-C₃)fluoroalkoxy, -NO₂, phenoxy, or (C₁-C₄)alkoxy, said alkyl being optionally mono- or di-substituted by a hydroxyl group; each of R₁ and R₂ is independently hydrogen or (C₁-C₃)alkyl; R' is hydrogen, halogen, (C₁-C₃)alkyl, or (C₁-C₄)alkoxy, with the proviso that R' is different from a methyl group at position 4 of the quinoline group; R" is hydrogen or (C₁-C₄)alkyl; n is 1, 2, or 3; and n' is 1 or 2.

In some embodiments, a compound of the invention is a compound of formula **(Id):** 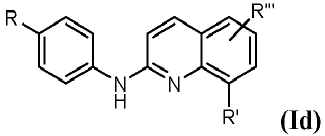 or a pharmaceutically acceptable salt thereof, wherein each of R and R' is independently as defined above and described in embodiments herein, both singly and in combination, and R'" is hydrogen or a group 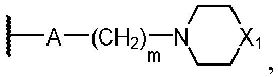 wherein A is O or NH, m is 2 or 3, and X₁ is O, CH₂ or N-CH₃.

In some embodiments, a compound of the invention is a compound of formula **(Id),** or a pharmaceutically acceptable salt thereof, wherein R is methyl, methoxy, trifluoromethyl, halogen, trifluoromethoxy, or amino; R' is halogen or methyl, and R'" is hydrogen or a group 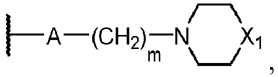 wherein A is O or NH, m is 2 or 3, and X₁ is O, CH₂ or N-CH₃. In some embodiments, R'" is hydrogen. In some embodiments, R'" is a group 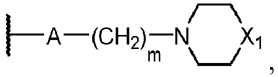 wherein A is O or NH, m is 2 or 3, and X₁ is O, CH₂ or N-CH₃. In some embodiments, R'" is a group 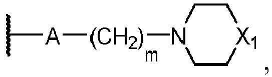 wherein A is O, m is 2 or 3, and X₁ is O, CH₂ or N-CH₃. In some embodiments, R'" is a group 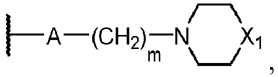 wherein A is NH, m is 2 or 3, and X₁ is O, CH₂ or N-CH₃.

The present invention moreover provides a **quinoline derivative** of formula (I') 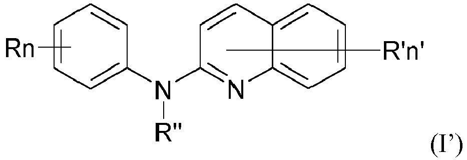 wherein:
R independently represent a halogen atom or a group chosen among a (C₁-C₃)fluoroalkyl group, a (C₁-C₃)fluoroalkoxy group, a -NR₁R₂ group, a (C₁-C₄)alkoxy group and a (C₁-C₃)alkyl group, said alkyl being optionally mono or di-substituted by a hydroxyl group,
n is 1 or 2,
n' is 1 or 2,
R₁ and R₂ are independently a hydrogen atom or a (C₁-C₃)alkyl group,
R' independently represent a halogen atom or a group chosen among a (C₁-C₃)alkyl group, a hydroxyl group, a -NR₁R₂ group, a morpholinyl or a morpholino group, a N-methylpiperazinyl group, a (C₁-C₃)fluoroalkyl group and a (C₁-C₄)alkoxy group, and can further be a group chosen among: 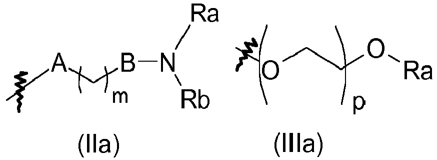
A is a covalent bond, an oxygen atom or NH,
B is a covalent bond or NH,
m is 2, 3 or 4,
p is 1, 2 or 3,
Ra and Rb independently represent a hydrogen atom, a (C₁-C₅)alkyl group or a (C₃-C₆)cycloalkyl group,
Ra and Rb can further form together with the nitrogen atom to which they are attached a saturated 5- or 6-membered heterocycle optionally containing a further heteroatom chosen among N, O and S, said heterocycle being optionally substituted by one or more Ra, provided that when R' is a group (IIa) or (IIIa), n' may be 2 only if the other R' groups is different from said group (IIa) or (IIIa), R" is a hydrogen atom or a (C₁-C₄)alkyl group,
or anyone of its metabolites or pharmaceutically acceptable salt or anyone of their enantiomers or diastereoisomers,
for use for treating or preventing cancer or dysplasia.

According to one aspect, the present invention provides a quinoline derivative of formula (I') or anyone of its metabolites or pharmaceutically acceptable salt or anyone of their enantiomers or diastereoisomers for use according to the present invention, wherein R independently represent a methyl group, a methoxy group, a trifluoromethyl group, a halogen atom and more particularly a fluorine or chlorine atom, a trifluoromethoxy group and an amino group.

According to a further aspect, herein is provided a quinoline derivative of formula (I') or anyone of its metabolites or pharmaceutically acceptable salt or anyone of their enantiomers or diastereoisomers for use according to the present invention, wherein R' independently represent a halogen atom and more particularly a fluorine or chlorine atom, a -NR₁R₂ group, and preferably an amino group, a hydroxyl group, a (C₁-C₃)alkyl group, preferably a methyl group, or a group 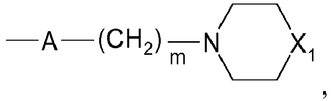 wherein A is O or NH, m is 2 or 3 and X₁ is O, CH₂ or N-CH₃, provided that when R' is such a group, n' is 1 or 2, and when n' is 2, the other R' group is different from said group.

According to a further aspect, herein is provided a quinoline derivative of formula (I') or anyone of its metabolites or pharmaceutically acceptable salt or anyone of their enantiomers or diastereoisomers for use according to the present invention, wherein R' independently represent a halogen atom and more particularly a fluorine or chlorine atom, a methyl group or a group 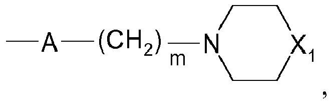 wherein A is O or NH, m is 2 or 3 and X₁ is O, CH₂ or N-CH₃, provided that when R' is such a group, n' is 1 or 2, and when n' is 2, the other R' group is different from said group.

According to a further aspect, herein is provided a quinoline derivative of formula (I') or anyone of its metabolites or pharmaceutically acceptable salt or anyone of their enantiomers or diastereoisomers for use according to the present invention, wherein n is 1, n' is 1 or 2, R" is H, R is selected from a methyl group, a methoxy group, a trifluoromethyl group, a halogen atom and more particularly a fluorine or chlorine atom, a trifluoromethoxy group and an amino group, and R' represents a halogen atom and more particularly a fluorine or chlorine atom, a methyl group or a group 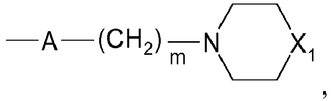 wherein A is O or NH, m is 2 or 3 and X₁ is O, CH₂ or N-CH₃, provided that when n' is 2, the other R' group is different from said group.

According to a further aspect, herein is provided a quinoline derivative of formula (I') or anyone of its metabolites or pharmaceutically acceptable salt or anyone of their enantiomers or diastereoisomers for use according to the present invention, wherein n is 1, n' is 1, R" is H, R is selected from a methyl group, a methoxy group, a trifluoromethyl group, a halogen atom and more particularly a fluorine or chlorine atom and a trifluoromethoxy group, and R' represents a halogen atom and more particularly a fluorine or chlorine atom or a methyl group.

According to a further aspect, herein is provided a quinoline derivative of formula (I') or anyone of its metabolites or pharmaceutically acceptable salt or anyone of their enantiomers or diastereoisomers for use according to the present invention, wherein it is defined by formula (I") 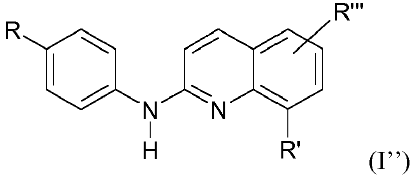 wherein
R is selected from a methyl group, a methoxy group, a trifluoromethyl group, a halogen atom and more particularly a fluorine or chlorine atom, a trifluoromethoxy group and an amino group, and
R' represents a halogen atom and more particularly a fluorine or chlorine atom or a methyl group, and
R'" represents a hydrogen atom or a group 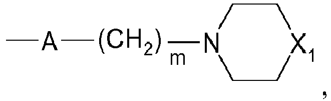 wherein A is O or NH, m is 2 or 3 and X₁ is O, CH₂ or N-CH₃, and in particular represents a hydrogen atom.

In some embodiments, a compound of the invention is a compound of formula: 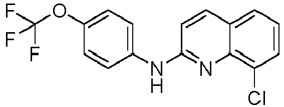 ("ABX464"), or a pharmaceutically acceptable salt thereof. In some embodiments, the compound ABX464, or a pharmaceutically acceptable salt thereof, is under an amorphous form. In some embodiments, the compound ABX464, or a pharmaceutically acceptable salt thereof, is under a crystallized form. In some embodiments, a crystallized form of the compound ABX464, or a pharmaceutically acceptable salt thereof, has a melting point at 120.5°C (± 2°C). In some embodiments, a crystallized form of the compound ABX464, or a pharmaceutically acceptable salt thereof, shows peaks in an x-ray powder diffractogram (XRPD) at angles 7.3, 14.6, 18.4, and 24.9. In some embodiments, a crystallized form of the compound ABX464, or a pharmaceutically acceptable salt thereof, shows one or more XRPD peaks at angles selected from 18.0, 24.2, 28.3, and 29.5. In some embodiments, a crystallized form of the compound ABX464, or a pharmaceutically acceptable salt thereof, shows one or more XRPD peaks at angles selected from 18.6, 22.3, 23.0, and 23.5.

In some embodiments, a compound of the invention is selected from **Table 1:**

**Table 1 (compounds of formula Ia as defined above)**

| | |
|---|---|
| **1** | 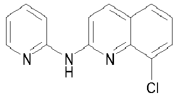 |
| **2** | 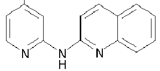 |
| **3** | 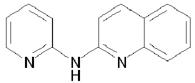 |
| **4** | 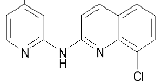 |
| **5** | 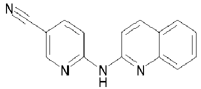 |
| **6** | 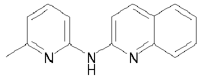 |
| **7** | 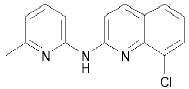 |
| **8** | 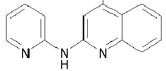 |
| **9** | 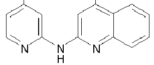 |
| **10** | 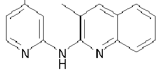 |
| **11** | 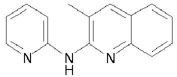 |
| **12** | 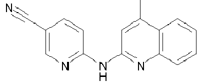 |
| **13** | 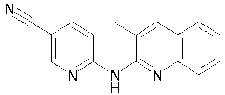 |
| **14** | 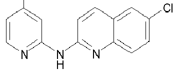 |
| **15** | 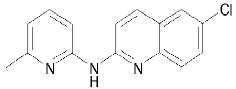 |
| **16** | 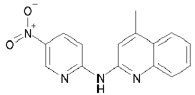 |
| **17** | 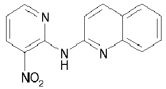 |
| **18** | 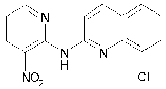 |
| **19** | 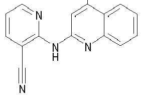 |
| **20** | 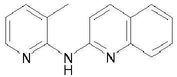 |
| **21** | 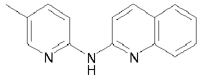 |
| **22** | 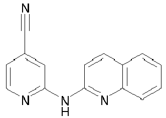 |
| **23** | 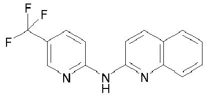 |
| **24** | 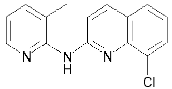 |
| **25** | 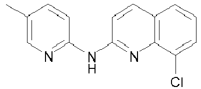 |
| **26** | 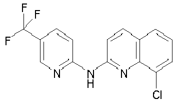 |
| **27** | 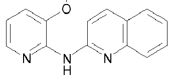 |
| **28** | 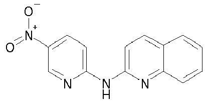 |
| **29** | 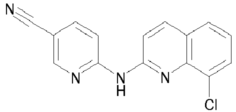 |
| **30** | 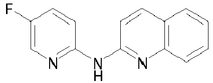 |
| **31** | 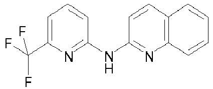 |
| **32** | 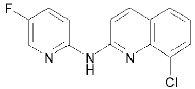 |
| **33** | 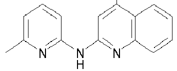 |
| **34** | 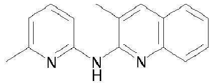 |
| **35** | 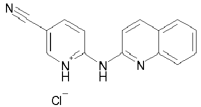 |
| **36** | 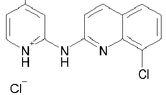 |
| **37** | 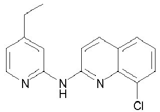 |
| **38** | 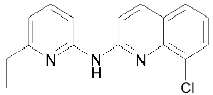 |
| **39** | 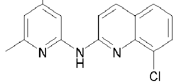 |
| **40** | 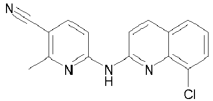 |
| **41** | 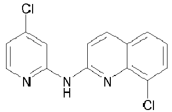 |
| **42** | 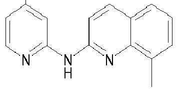 |
| **43** | 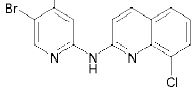 |
| **44** | 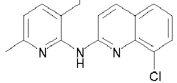 |
| **45** | 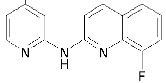 |
| **46** | 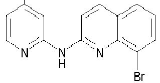 |
| **47** | 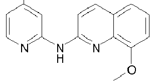 |
| **48** | 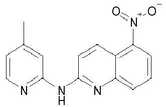 |
| **49** | 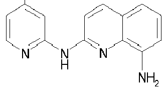 |
| **50** | 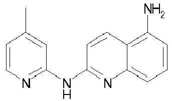 |
| **51** | 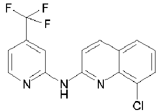 |
| **52** | 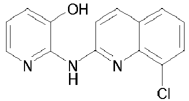 |
| **53** | 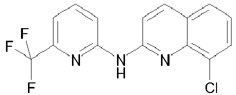 |
| **54** | 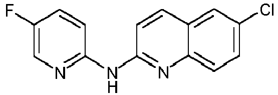 |
| **55** | 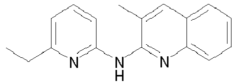 |
| **56** | 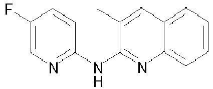 |
| **57** | 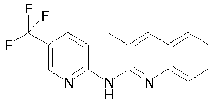 |
| **58** | 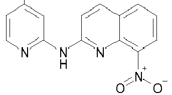 |
| **59** | 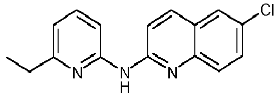 |
| **60** | 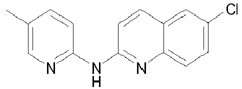 |
| **61** | 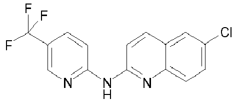 |
| **62** | 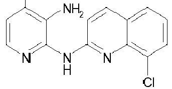 |
| **63** | 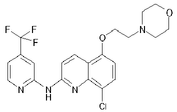 |
| **64** | 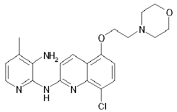 |
| **65** | 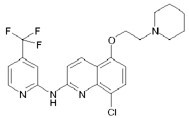 |
| **66** | 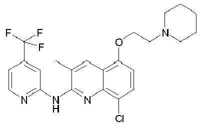 |
| **67** | 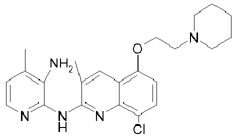 |
| **68** | 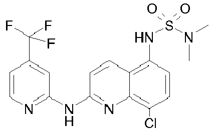 |
| **69** | 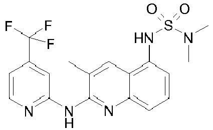 |
| **70** | 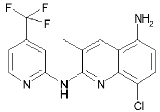 |
| **71** | 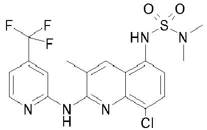 |
| **72** | 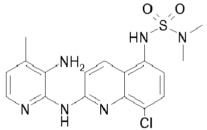 |
| **73** | 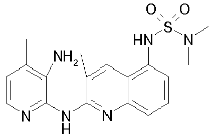 |
| **74** | 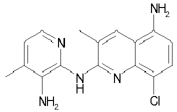 |
| **75** | 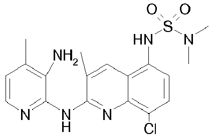 |
| **76** | 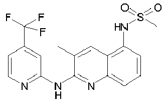 |
| **77** | 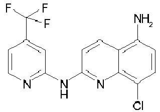 |
| **78** | 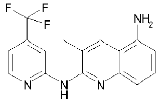 |
| **79** | 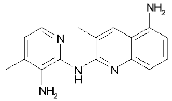 |
| **80** | 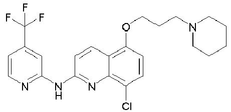 |
| **81** | 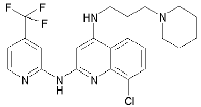 |
| **82** | 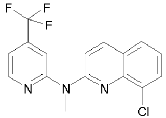 |
| **83** | 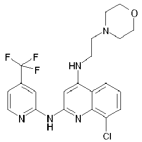 |
| **84** | 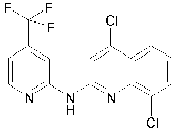 |
| **85** | 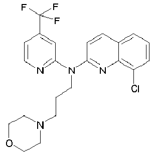 |
| **86** | 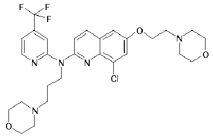 |
| **87** | 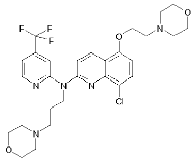 |
| **88** | 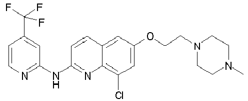 |
| **89** | 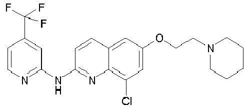 |
| **90** | 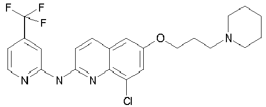 |
| **91** | 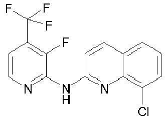 |
| **92** | 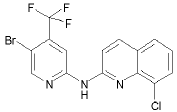 |
| **93** | 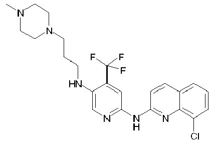 |
| **94** | 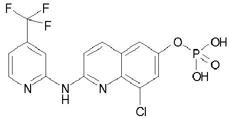 |

or a pharmaceutically acceptable salt thereof.

In some embodiments, a compound of the invention is selected from **Table 2:**

**Table 2 (compounds of formula Ib as defined above)**

| | |
|---|---|
| **95** | 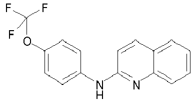 |
| **96** | 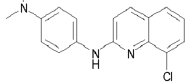 |
| **97** | 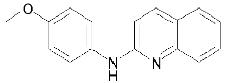 |
| **98** | 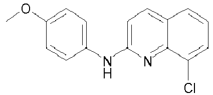 |
| **99** | 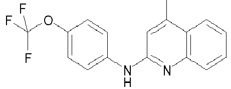 |
| **100** | 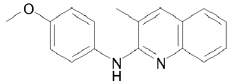 |
| **101** | 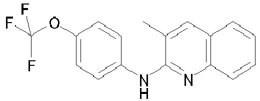 |
| **102** | 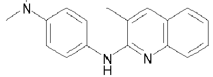 |
| **103** | 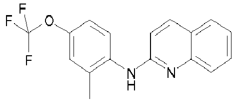 |
| **104** | 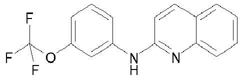 |
| **105** | 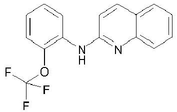 |
| **106** | 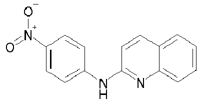 |
| **107** | 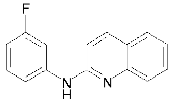 |
| **108** | 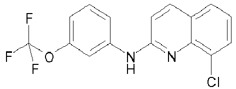 |
| **109** | 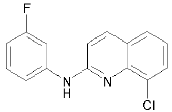 |
| **110** | 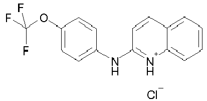 |
| **111** | 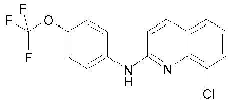 |
| **112** | 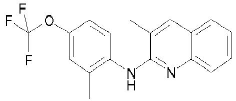 |
| **113** | 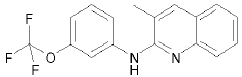 |
| **114** | 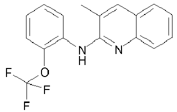 |
| **115** | 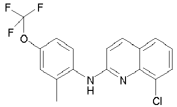 |
| **116** | 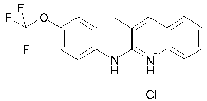 |
| **117** | 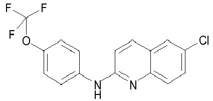 |
| **118** | 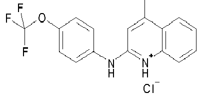 |
| **119** | 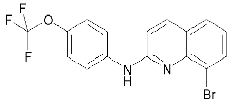 |
| **120** | 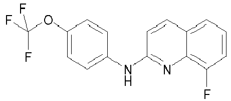 |
| **121** | 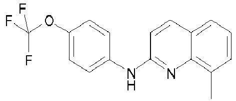 |
| **122** | 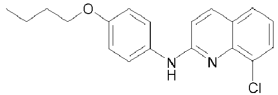 |
| **123** | 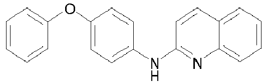 |
| **124** | 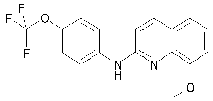 |
| **125** | 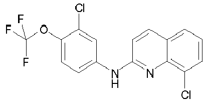 |
| **126** | 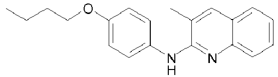 |
| **127** | 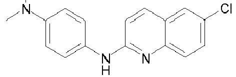 |
| **128** | 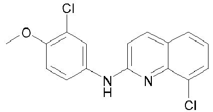 |
| **129** | 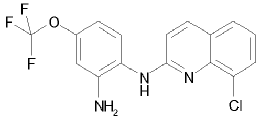 |
| **130** | 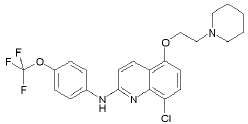 |
| **131** | 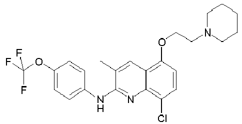 |
| **132** | 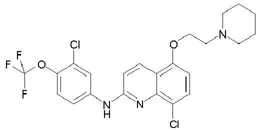 |
| **133** | 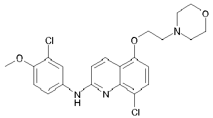 |
| **134** | 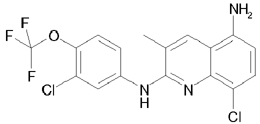 |
| **135** | 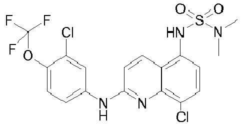 |
| **136** | 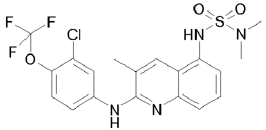 |
| **137** | 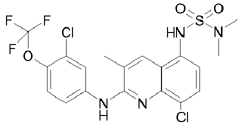 |
| **138** | 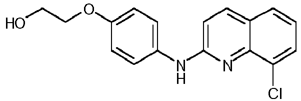 |
| **139** | 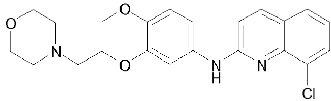 |
| **140** | 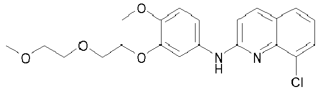 |
| **141** | 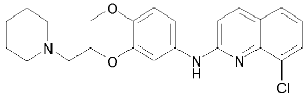 |
| **142** | 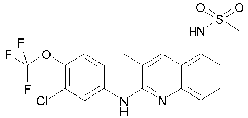 |
| **143** | 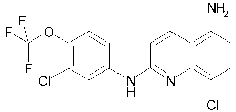 |
| **144** | 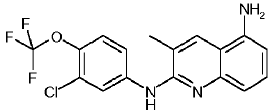 |
| **145** | 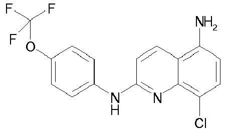 |
| **146** | 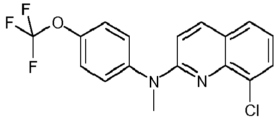 |
| **147** | 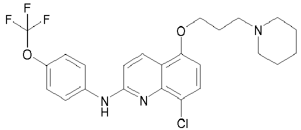 |
| **148** | 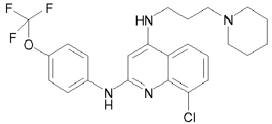 |
| **149** | 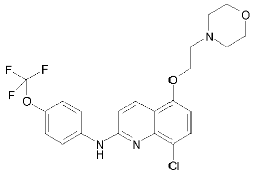 |
| **150** | 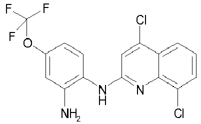 |
| **151** | 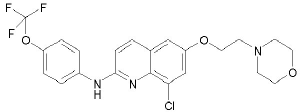 |
| **152** | 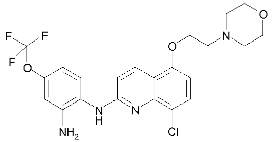 |
| **153** | 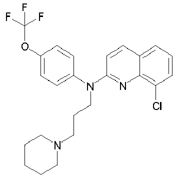 |
| **154** | 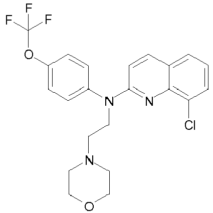 |
| **155** | 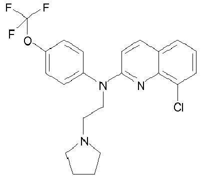 |
| **156** | 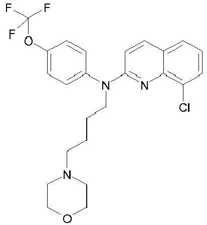 |
| **157** | 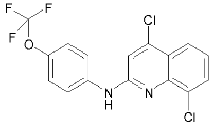 |
| **158** | 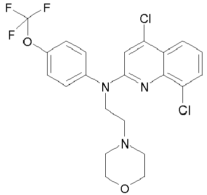 |
| **159** | 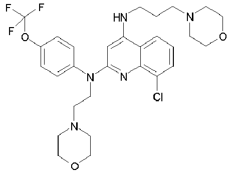 |
| **160** | 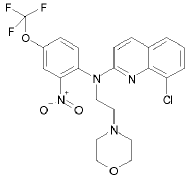 |
| **161** | 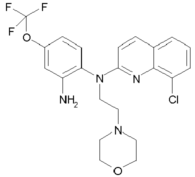 |
| **162** | 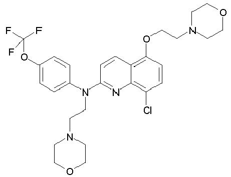 |
| **163** | 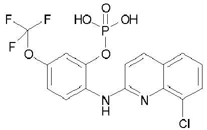 |
| **164** | 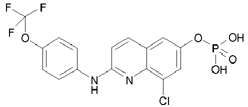 |

or a pharmaceutically acceptable salt thereof.

In some embodiments, a compound of the invention is selected from **Table 3:**

**Table 3 (compounds of formula (I) other that compounds (Ia) and (Ib))**

| | |
|---|---|
| **165** | 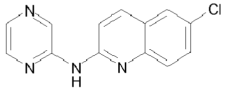 |
| **166** | 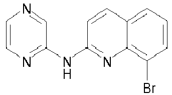 |
| **167** | 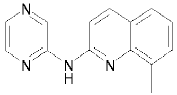 |
| **168** | 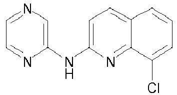 |
| **169** | 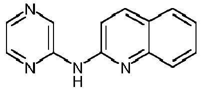 |
| **170** | 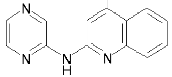 |
| **171** | 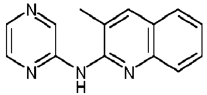 |
| **172** | 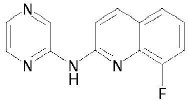 |
| **173** | 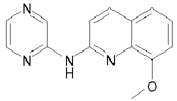 |
| **174** | 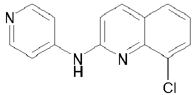 |
| **175** | 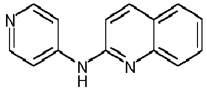 |
| **176** | 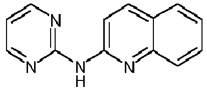 |
| **177** | 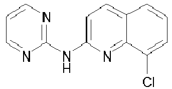 |
| **178** | 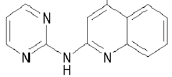 |
| **179** | 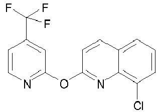 |
| **180** | 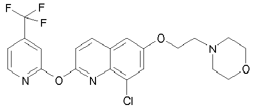 |
| **181** | 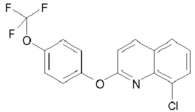 |
| **182** | 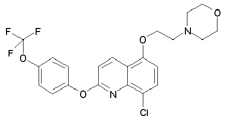 |
| **183** | 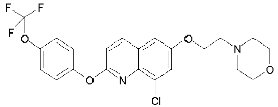 |

or a pharmaceutically acceptable salt thereof.

In some embodiments, a compound described herein is in a salt form selected from sulfate, hydrobromide, citrate, trifluoroacetate, ascorbate, hydrochloride, tartrate, triflate, maleate, mesylate, formate, acetate, fumarate, and sulfonate. In some embodiments, a compound described herein is in salt form as alkylsufonate or arylsulfonate. In some embodiments, a compound described herein is in salt form as mesylate, triflate, edisylate, besylate and tosylate.

In one aspect, the present invention provides a metabolite of a compound described herein. In some embodiments, the present invention provides a N-glucuronide metabolite of a compound described herein.

In some embodiments, a compound of the invention is a compound of formula **(IV):** 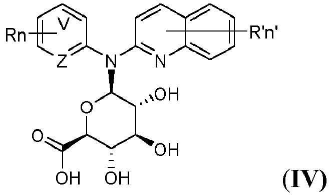 or a pharmaceutically acceptable salt thereof, wherein each of variables V, Z, R, R', n, and n' is as defined above and described in embodiments herein, both singly or in combination.

In some embodiments, a compound of the invention is a compound of formula **(IVa):** 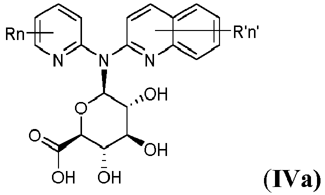 or a pharmaceutically acceptable salt thereof, wherein each of variables R, R', n, and n' is independently as defined above and described in embodiments herein, both singly and in combination.

In some embodiments, a compound of the invention is a compound of formula **(IVb):** 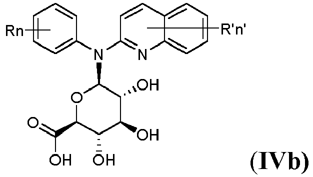 or a pharmaceutically acceptable salt thereof, wherein each of variables R, R', n, and n' is independently as defined above and described in embodiments herein, both singly and in combination.

In some embodiments, a compound of the invention is a compound of formula **(IVc):** 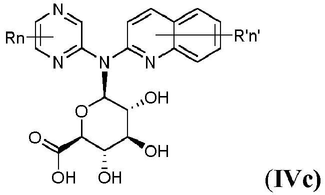 or a pharmaceutically acceptable salt thereof, wherein each of variables R, R', n, and n' is independently as defined above and described in embodiments herein, both singly and in combination.

In some embodiments, a compound of the invention is a compound of formula **(IVb'):** 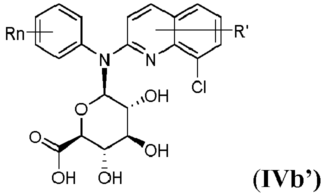 or a pharmaceutically acceptable salt thereof, wherein each of variables R, R', and n is independently as defined above and described in embodiments herein, both singly and in combination.

In some embodiments, a compound of the invention is a compound of formula (**IVd**): 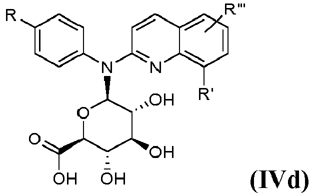 or a pharmaceutically acceptable salt thereof, wherein each of variables R, R', R'" is independently as defined above and described in embodiments herein, both singly and in combination.

In some embodiments, the present invention provides a method for treating an inflammatory disease, disorder or condition comprising administering to a patient in need thereof a compound of any one of formulas **(IV), (IVa), (IVb), (IVc),** (**IVb**') and **(IVd),** or a pharmaceutically acceptable salt thereof.

In some embodiments, a compound of the invention is a compound of formula: 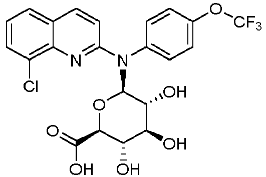 or a pharmaceutically acceptable salt thereof.

The compounds of the invention may exist in the form of free bases or of addition salts with pharmaceutically acceptable acids. Suitable physiologically acceptable acid addition salts of compounds of the present invention include sulfate, hydrobromide, citrate, trifluoroacetate, ascorbate, hydrochloride, triflate, tartrate, maleate, formate, acetate, fumarate and sulfonate, in particular alkylsufonate or arylsulfonate, and more particularly mesylate, triflate, edisylate, besylate and tosylate.

The compounds of the present invention and or salts thereof may form solvates or hydrates and the invention includes all such solvates and hydrates. The terms "hydrates" and "solvates" simply mean that the compounds according to the invention can be in the form of a hydrate or solvate, i.e. combined or associated with one or more water or solvent molecules. This is only a chemical characteristic of such compounds, which can be applied for all organic compounds of this type.

The compounds of of the present invention can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

In the context of the present invention, the term:
- "halogen" is understood to mean chlorine, fluorine, bromine, or iodine, and in particular denotes chlorine, fluorine or bromine,
- "(C₁-C₅)alkyl" as used herein respectively refers to C₁-C₅ normal, secondary or tertiary saturated hydrocarbon. Examples are, but are not limited to, methyl, ethyl, 1-propyl, 2-propyl, butyl, pentyl,
- "(C₃-C₆)cycloalkyl" as used herein respectively refers to cyclic saturated hydrocarbon. Examples are, but are not limited to cyclopropyl, cyclobutyl, cyclopentyl, cyclohexyl,
- "(C₁-C₄)alkoxy" as used herein respectively refers to O-(C₁-C₄)alkyl moiety, wherein alkyl is as defined above. Examples are, but are not limited to, methoxy, ethoxy, 1-propoxy, 2-propoxy, butoxy,
- "fluoroalkyl group" and "fluoroalkoxy group" refers respectively to alkyl group and alkoxy group as above-defined, said groups being substituted by at least one fluorine atom. Examples are perfluoroalkyl groups, such as trifluoromethyl or perfluoropropyl,
- "saturated 5- or 6-membered heterocycle" as used herein respectively refers to a saturated cycle comprising at least one heteroatom. Examples are, but are not limited to, morpholine, piperazine, thiomorpholine, piperidine, pyrrolidine.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference.

Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention.

### Cancers

A compound or anyone of its metabolites or pharmaceutically acceptable salts thereof, as defined above, may be useful in the treatment or prevention of various cancers.

As used herein the term "cancer", and unless stated otherwise, may relate to any disorder associated with abnormal cell growth, which thus includes malignant tumors and benign tumors, metastatic tumors and non-metastatic tumors, solid tumors and non-solid tumors, such as Blood-Related Cancers which may thus include Leukaemia, Lymphoma and Myeloma; it may also relate to Central Nervous System (CNS) cancers and non-CNS cancers. Unless stated otherwise, the term "*cancer*" also encompasses juvenile and non-juvenile cancers, Recurrent and Non-Recurrent cancers as well as cancer relapses.

When the considered cancer is a Blood-Related Cancer, it may be selected from; small lymphocytic lymphoma, non-Hodgkin's lymphoma, indolent non-Hodgkin's lymphoma (iNHL), refractory iNHL, mantle cell lymphoma, follicular lymphoma, lymphoplasmacytic lymphoma, marginal zone lymphoma, immunoblastic large cell lymphoma, lymphoblastic lymphoma, Splenic marginal zone B-cell lymphoma (+/-villous lymphocytes), nodal marginal zone lymphoma (+/-monocytoid B-cells), extranodal marginal zone B-cell lymphoma of mucosa-associated lymphoid tissue type, cutaneous T-cell lymphoma, extranodal T-cell lymphoma, anaplastic large cell lymphoma, angioimmunoblastic T-cell lymphoma, mycosis fungoides, B-cell lymphoma, diffuse large B-cell lymphoma, Mediastinal large B-cell lymphoma, Intravascular large B-cell lymphoma, Primary effusion lymphoma, small non-cleaved cell lymphoma, Burkitt's lymphoma, multiple myeloma, plasmacytoma, acute lymphocytic leukemia, T-cell acute lymphoblastic leukemia, B-cell acute lymphoblastic leukemia, B-cell prolymphocytic leukemia, acute myeloid leukemia, chronic lymphocytic leukemia, juvenile myelomonocytic leukemia, minimal residual disease, hairy cell leukemia, primary myelofibrosis, secondary myelofibrosis, chronic myeloid leukemia, myelodysplastic syndrome, myeloproliferative disease, or Waldestrom's macroglobulinemia.

In other variations, the cancer is pancreatic cancer, urological cancer, bladder cancer, colorectal cancer, colon cancer, breast cancer, prostate cancer, renal cancer, hepatocellular cancer, thyroid cancer, gall bladder cancer, lung cancer (e.g. non-small cell lung cancer, small-cell lung cancer), ovarian cancer, cervical cancer, gastric cancer, endometrial cancer, oesophageal cancer, head and neck cancer, melanoma, neuroendocrine cancer, CNS cancer, brain tumors (e.g., glioma, anaplastic oligodendroglioma, adult glioblastoma multiforme, and adult anaplastic astrocytoma), bone cancer, soft tissue sarcoma, retinoblastomas, neuroblastomas, peritoneal effusions, malignant pleural effusions, mesotheliomas, Wilms tumors, trophoblastic neoplasms, hemangiopericytomas, Kaposi's sarcomas, myxoid carcinoma, round cell carcinoma, squamous cell carcinomas, oesophageal squamous cell carcinomas, oral carcinomas, cancers of the adrenal cortex, or ACTH-producing tumors.

According to one embodiment, the cancer is selected from head and neck cancer, Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bladder Cancer, Bone Cancer, brain cancer, Brain and Spinal Cord Tumors in Adults, Brain and Spinal Cord Tumors in Children, Anaplastic astrocytomas, Breast cancer, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Cancer, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), Colorectal Cancer, colorectal adenoma, Cutaneous Squamous Cell Carcinoma, Endometrial Cancer, Epithelial Ovarian Cancer, Epithelial Ovarian Cancer associated with metastasis, oesophageal cancer, Oesophagus Squamous Cell Carcinoma, Ewing sarcoma, Ewing Family of Tumors, Lymphoblastic leukaemia (ALL), Eye Cancer, such as Ocular Melanoma and Lymphoma, Gallbladder Cancer, Gastric Cancer, gastrointestinal cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Kidney Cancer, Laryngeal and Hypopharyngeal Cancer, Leiomyosarcoma, Leukemia, Leukemia in Children, Liver Cancer, Lung Cancer, Lung Carcinoid Tumor, Lymphoma, Lymphoma of the Skin, Malignant Mesothelioma, Mantle cell lymphoma, Medulloblastoma, Melanoma Skin Cancer, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Non-Small Cell Lung Cancer, Gefitinib-resistant non-small cell lung cancer, Oral cancer, Oral Cavity and Oropharyngeal Cancer, Osteosarcoma, Pulmonary Metastatic Osteosarcoma, Ovarian Cancer, Pancreatic Cancer, thyroid carcinoma, Papillary Thyroid Carcinoma, Pediatric Spinal Ependymoma, Penile Cancer, Pituitary Tumors, Pituitary Adenoma, Proneural tumors, Prostate Cancer, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Stomach Cancer, Testicular Cancer, Thymus Cancer, Thyroid Cancer, Uterine Sarcoma, Vaginal Cancer, Vulvar Cancer, Renal cancer, Retinoblastoma, Waldenstrom Macroglobulinemia and Wilms Tumor.

According to one particular embodiment, herein is further provided a compound or anyone of its metabolites or pharmaceutically acceptable salts, as defined herein, and in particular 8-Chloro-N-(4-(trifluoromethoxy)phenyl)quinolin-2-amine for use in the prevention or treatment of cancer selected from Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bone Cancer, brain cancer, Brain and Spinal Cord Tumors in Adults, Brain and Spinal Cord Tumors in Children, Anaplastic astrocytomas, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), colorectal adenoma, Cutaneous Squamous Cell Carcinoma, Endometrial Cancer, Epithelial Ovarian Cancer, Epithelial Ovarian Cancer associated with metastasis, Oesophagus Squamous Cell Carcinoma, Ewing sarcoma, Ewing Family of Tumors, Lymphoblastic leukaemia (ALL), Eye Cancer, such as Ocular Melanoma and Lymphoma, Gastric Cancer, gastrointestinal cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Laryngeal and Hypopharyngeal Cancer, Leiomyosarcoma, Leukemia, Leukemia in Children, Lung Carcinoid Tumor, Lymphoma, Lymphoma of the Skin, Malignant Mesothelioma, Mantle cell lymphoma, Medulloblastoma, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Gefitinib-resistant non-small cell lung cancer, Oral cancer, Oral Cavity and Oropharyngeal Cancer, Osteosarcoma, Pulmonary Metastatic Osteosarcoma, thyroid carcinoma, Papillary Thyroid Carcinoma, Pediatric Spinal Ependymoma, Penile Cancer, Pituitary Tumors, Pituitary Adenoma, Proneural tumors, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Testicular Cancer, Thymus Cancer, Uterine Sarcoma, Vaginal Cancer, Vulvar Cancer, Renal cancer, Retinoblastoma, Waldenstrom Macroglobulinemia and Wilms Tumor.

According to one embodiment, a compound or anyone of its metabolites or pharmaceutically acceptable salts, as defined herein, and in particular 8-Chloro-N-(4-(trifluoromethoxy)phenyl)quinolin-2-amine also named (8-chloro-quinoline-2-yl)-(4-trifluoromethoxy-phenyl)-amine, are more particularly dedicated to treat or prevent any metastasis of any of the above cancers in the brain, bones, liver, lung, kidney, muscles, abdomen, or other tissues.

According to one embodiment, a compound or anyone of its metabolites or pharmaceutically acceptable salts, as described herein, and in particular 8-Chloro-N-(4-(trifluoromethoxy)phenyl)quinolin-2-amine also named (8-chloro-quinoline-2-yl)-(4-trifluoromethoxy-phenyl)-amine, are more particularly dedicated to treat or prevent the following cancers: Head and neck cancer, stomach cancer, Breast cancer, basal and squamous skin cell cancer, liver cancer, brain cancer, lung cancer, pancreatic cancer, eye cancer, gastrointestinal cancer, colorectal cancer, bladder cancer, bone cancer and renal cancer.

According to one particular embodiment, a compound or anyone of its metabolites or pharmaceutically acceptable salts thereof, as described herein, and in particular 8-Chloro-N-(4-(trifluoromethoxy)phenyl)quinolin-2-amine also named (8-chloroquinoline-2-yl)-(4-trifluoromethoxy-phenyl)-amine, are more particularly dedicated to treat or prevent the following cancers: Nasopharyngeal carcinoma (recurrence or metastasis), Astrocytic gliomas, cancer, Prostate cancer, Non-small Cell Lung Cancer (NSCLC), Cholangiocarcinoma (lymph node involvement and distant metastasis), Bladder cancer, Neck squamous cell carcinoma (HNSCC), Oral cancer, Ewing Sarcoma, metastatic Ewing sarcoma, Gefitinib-resistant non-small cell lung cancer, Cutaneous squamous cell carcinoma, Osteosarcoma, Malignant melanoma, Osteosarcoma associated with pulmonary metastasis, Colorectal carcinoma, Breast cancer, Colorectal Cancer (CRC), Gastric cancer (GC), Glioblastoma multiforme, Glioma, Glioblastoma, Retinoblastoma, Nasopharyngeal carcinoma (NPC), Colorectal cancer, Bladder cancer, Acute myeloid leukemia, Astrocytomas (grade I-II-III-IV), B- or NK/T-cell lymphomas, Bladder cancer (advanced malignancy), Gastric dysplasia, Gastric cancer associated with lymph node metastasis, Glioma, Myelodysplastic Syndrome, Pancreatic ductal adenocarcinoma (associated with metastasis to Lymph node and/or tumour node metastasis), HER2 positive breast cancer, Lung adenocarcinoma, Medulloblastoma, Non-small cell lung cancer (associated with metastasis to Lymph node and/or tumour node metastasis), Breast cancer (associated with tumor node metastasis and lymph node metastasis), Endometrial cancer, Medulloblastoma, Cutaneous squamous cell carcinoma, Intrahepatic cholangiocarcinoma (ICC), Gallbladder cancer, Colorectal adenoma (CRA), Acute lymphoblastic leukemia (ALL), Glioblastoma multiforme (GBM), Pancreatic ductal adenocarcinoma (PDAC), Bladder cancer (BC), Ovarian cancer (OC), Leukemia, Myelodysplastic syndrome (MDS), Proneural tumors, HNSCC (Head and Neck Squamous cell carcinoma, primary tumor, Renal cell carcinoma (RCC), Uveal melanoma, , Malignant prostate cancer, Oral squamous cell carcinomas (OSCC), Anaplastic astrocytomas, Oesophageal cancer, Astrocytoma, Cholangiocarcinoma, Breast cancer (associated with lymph node metastasis), Pediatric spinal ependymomas, Pancreatic carcinoma, Epithelial ovarian cancer, Breast cancer (associated with bone metastasis), Hepatocarcinoma, Myelodysplastic syndrome, Malignant melanoma (node metastasis), Neuroendocrine neoplasms of the small intestine (SI-NENs), Epithelial ovarian cancer (associated with metastasis to lymph nodes, peritoneum, and distant organs), Myelodysplastic syndrome, Pediatric ependymoma, Gastric carcinoma, Breast cancer (ER-alpha positive), Hepatocellular carcinoma (HCC), Ovarian cancer, Oesophageal squamous cell carcinoma (ESCC), Invasive breast ductal carcinoma (associated with lymph node metastasis), Lymphoma, Myeloma (MM), Lymphoblastic leukaemia (ALL), Chronic lymphocytic leukaemia (CLL), Acute myeloid leukaemia (AML), Non-Hodgkin's lymphoma (NHL), B- or NK/T-cell lymphomas, Cervical carcinoma, Carcinosarcoma, Cervical cancer, Cervical intraepithelial neoplasia (CIN2 and CIN3 grade), Metachronous gastric cancer, Clear cell renal cell carcinoma, Myelodysplastic syndrome (MDS), Bladder cancer (bca), Pancreatic cancer, Ccrccs (clear cell renal cell carcinomas), Ulcerative colitis (UC)-neoplastic tissues (Colitis-associated cancer (CAC), dysplasia and sporadic colorectal cancer (S-CRC)), Cervical intraepithelial neoplasia, Head and neck cancer.

According to one further embodiment, the cancer is selected from head and neck cancer, Head and Neck Squamous Cell Carcinoma, Neck Squamous Cell Carcinoma, Malignant melanoma, stomach cancer, Breast cancer, Breast cancer in Women, Breast Cancer in Young Women, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, eye cancer, Gastric Cancer, gastrointestinal cancer, Astrocytic Glioma, Astrocytoma (grade I, II, III, or IV), colorectal cancer, colorectal adenoma, Cutaneous Squamous Cell Carcinoma, bladder cancer, bone cancer, Recurrent Breast Cancer, Hereditary Breast Cancer, HER2 positive Breast Cancer, Breast Cancer associated with lymph node metastatis, ER-alpha positive Breast Cancer, renal cancer, Cervical Intraepithelial Neoplasia, Cholangiocarcinoma, Leiomyosarcoma, Chronic Lymphocytic Leukemia (CLL), Chronic Myeloid Leukemia (CML), Chronic Myelomonocytic Leukemia (CMML), Acute Myeloid Leukemia (AML) in adults or children, Acute Lymphoblastic Leukemia,, B- or NK/T-cell lymphomas, cervical cancer, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, kidney cancer, Endometrial cancer, ovarian cancer, Epithelial Ovarian Cancer, Epithelial Ovarian Cancer associated with metastasis, Oesophageal cancer, Oesophageal Squamous Cell Carcinoma, Ewing sarcoma, Lymphoblastic leukaemia (ALL), Mantle cell lymphoma, Medulloblastoma, Lymphoma, Myelodysplastic syndrome, Meningioma, Multiple Myeloma (MM), Multiple Myeloma with Osteonecrosis of the Jaw, Nasopharyngeal Cancer, recurrent or metastatic Nasopharyngeal carcinoma, Neuroblastoma, Neuroglioma, Papillary Thyroid Carcinoma, Pediatric Spinal Ependymoma, Osteosarcoma, Pulmonary Metastatic Osteosarcoma, pancreatic cancer, thyroid carcinoma, sarcoma, pituitary tumors, Pituitary Adenoma, Proneural tumors, Squamous Cell Carcinoma of the Tongue, Retinoblastoma and prostate cancer.

According to a further embodiment of the present invention, the cancer is selected from Head and Neck cancer, Head and Neck Squamous Cell Carcinoma, Neck Squamous Cell Carcinoma, malignant melanoma, Astrocytic Glioma, Glioma, stomach cancer, Breast cancer, Cholangiocarcinoma, recurrent or metastatic Nasopharyngeal carcinoma, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, Glioblastoma, osteosarcoma, Pulmonary Metastatic Osteosarcoma, pancreatic cancer, eye cancer, gastrointestitnal cancer, colorectal cancer, colorectal adenoma, Cutaneous Squamous Cell Carcinoma, Endometrial cancer, Epithelial Ovarian Cancer, oesophageal cancer, Ewing sarcoma, gastric cancer, Hepatocellular carcinoma, HER2 positive Breast Cancer, bladder cancer, bone cancer, prostate cancer, Retinoblastoma and renal cancer.

In a particular embodiment, the cancers which are particularly considered are selected from: Anaplastic astrocytomas, Astrocytic gliomas, Bladder cancer, Breast cancer, Cholangiocarcinoma, Colorectal cancer, Colorectal adenoma, Cutaneous squamous cell carcinoma, Endometrial cancer, Epithelial ovarian cancer, Esophageal cancer, Ewing sarcoma, Gastric cancer, Gefitinib-resistant non-small cell lung cancer , Glioblastoma, Glioma, Hepatocellular carcinoma, HER2 positive breast cancer, Head and Neck Squamous Cell Carcinoma, Malignant melanoma, Nasopharyngeal carcinoma (recurrence or metastasis), Neck squamous cell carcinoma, Non-small cell lung cancer, Oral cancer, Osteosarcoma, Osteosarcoma associated with pulmonary metastasis, Prostate cancer and Retinoblastoma.

According to one aspect, the present invention relates to 8-Chloro-N-(4-(trifluoromethoxy)phenyl)quinolin-2-amine for use in the prevention and/or treatment of cancer, in particular selected from head and neck cancer, stomach cancer, Breast cancer, basal and squamous skin cell cancer, liver cancer, brain cancer, lung cancer, eye cancer, gastrointestitnal cancer, colorectal cancer, bladder cancer, bone cancer, renal cancer, Chronic Lymphocytic Leukemia (CLL), Chronic Myeloid Leukemia (CML), Chronic Myelomonocytic Leukemia (CMML), Acute Myeloid Leukemia (AML), B- or NK/T-cell lymphomas, cervical cancer, kidney cancer, Endometrial cancer, ovarian cancer, Oesophageal cancer, Lymphoblastic leukaemia (ALL), Lymphoma, Myelodysplastic syndrome, Multiple Myeloma (MM), Nasopharyngeal Cancer, Neuroblastoma, Osteosarcoma, Pulmonary Metastatic Osteosarcoma, pancreatic cancer, thyroid carcinoma, sarcoma, pituitary tumors and prostate cancer.

According to a particular embodiment, 8-Chloro-N-(4-(trifluoromethoxy)phenyl)quinolin-2-amine may be useful in the treatment or prevention of head and neck cancer, stomach cancer, Breast cancer, basal and squamous skin cell cancer, liver cancer, brain cancer, lung cancer, eye cancer, gastrointestinal cancer, colorectal cancer, bladder cancer, bone cancer or renal cancer.

Cancer includes, in one embodiment, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythro leukemia, chronic leukemia, chronic myelocytic leukemia, chronic lymphocytic leukemia), polycythemia vera, lymphoma (e.g., Hodgkin's disease or non-Hodgkin's disease), Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, and retinoblastoma).

In some embodiments, the cancer is glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, or retinoblastoma.

In some embodiments, the cancer is acoustic neuroma, astrocytoma (e.g. Grade I - Pilocytic Astrocytoma, Grade II - Low-grade Astrocytoma, Grade III - Anaplastic Astrocytoma, or Grade IV - Glioblastoma (GBM)), chordoma, CNS lymphoma, craniopharyngioma, brain stem glioma, ependymoma, mixed glioma, optic nerve glioma, subependymoma, medulloblastoma, meningioma, metastatic brain tumor, oligodendroglioma, pituitary tumors, primitive neuroectodermal (PNET) tumor, or schwannoma. In some embodiments, the cancer is a type found more commonly in children than adults, such as brain stem glioma, craniopharyngioma, ependymoma, juvenile pilocytic astrocytoma (JPA), medulloblastoma, optic nerve glioma, pineal tumor, primitive neuroectodermal tumors (PNET), or rhabdoid tumor. In some embodiments, the patient is an adult human. In some embodiments, the patient is a child or pediatric patient.

Cancer includes, in another embodiment, without limitation, mesothelioma, hepatobilliary (hepatic and billiary duct), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, non-Hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

In some embodiments, the cancer is selected from hepatocellular carcinoma, ovarian cancer, ovarian epithelial cancer, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical adenoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from hepatocellular carcinoma (HCC), hepatoblastoma, colon cancer, rectal cancer, ovarian cancer, ovarian epithelial cancer, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical adenoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, the present invention provides a method for treating a cancer that presents as a solid tumor, such as a sarcoma, carcinoma, or lymphoma, comprising the step of administering a disclosed compound, or a pharmaceutically acceptable salt thereof, to a patient in need thereof. Solid tumors generally comprise an abnormal mass of tissue that typically does not include cysts or liquid areas. In some embodiments, the cancer is selected from renal cell carcinoma, or kidney cancer; hepatocellular carcinoma (HCC) or hepatoblastoma, or liver cancer; melanoma; breast cancer; colorectal carcinoma, or colorectal cancer; colon cancer; rectal cancer; anal cancer; lung cancer, such as non-small cell lung cancer (NSCLC) or small cell lung cancer (SCLC); ovarian cancer, ovarian epithelial cancer, ovarian carcinoma, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical carcinoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from renal cell carcinoma, hepatocellular carcinoma (HCC), hepatoblastoma, colorectal carcinoma, colorectal cancer, colon cancer, rectal cancer, anal cancer, ovarian cancer, ovarian epithelial cancer, ovarian carcinoma, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, chondrosarcoma, anaplastic thyroid cancer, adrenocortical carcinoma, pancreatic cancer, pancreatic ductal carcinoma, pancreatic adenocarcinoma, glioma, brain cancer, neurofibromatosis-1 associated malignant peripheral nerve sheath tumors (MPNST), Waldenstrom's macroglobulinemia, or medulloblastoma.

In some embodiments, the cancer is selected from hepatocellular carcinoma (HCC), hepatoblastoma, colon cancer, rectal cancer, ovarian cancer, ovarian epithelial cancer, ovarian carcinoma, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical carcinoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, the cancer is hepatocellular carcinoma (HCC). In some embodiments, the cancer is hepatoblastoma. In some embodiments, the cancer is colon cancer. In some embodiments, the cancer is rectal cancer. In some embodiments, the cancer is ovarian cancer, or ovarian carcinoma. In some embodiments, the cancer is ovarian epithelial cancer. In some embodiments, the cancer is fallopian tube cancer. In some embodiments, the cancer is papillary serous cystadenocarcinoma. In some embodiments, the cancer is uterine papillary serous carcinoma (UPSC). In some embodiments, the cancer is hepatocholangiocarcinoma. In some embodiments, the cancer is soft tissue and bone synovial sarcoma. In some embodiments, the cancer is rhabdomyosarcoma. In some embodiments, the cancer is osteosarcoma. In some embodiments, the cancer is anaplastic thyroid cancer. In some embodiments, the cancer is adrenocortical carcinoma. In some embodiments, the cancer is pancreatic cancer, or pancreatic ductal carcinoma. In some embodiments, the cancer is pancreatic adenocarcinoma. In some embodiments, the cancer is glioma. In some embodiments, the cancer is malignant peripheral nerve sheath tumors (MPNST). In some embodiments, the cancer is neurofibromatosis-1 associated MPNST. In some embodiments, the cancer is Waldenstrom's macroglobulinemia. In some embodiments, the cancer is medulloblastoma.

The present invention further features methods and compositions for the diagnosis, prognosis and treatment of viral-associated cancers, including human immunodeficiency virus (HIV) associated solid tumors, human papilloma virus (HPV)-16 positive incurable solid tumors, and adult T-cell leukemia, which is caused by human T-cell leukemia virus type I (HTLV-I) and is a highly aggressive form of CD4+ T-cell leukemia characterized by clonal integration of HTLV-I in leukemic cells (See https://clinicaltrials.gov/ct2/show/study/ NCT02631746); as well as virus-associated tumors in gastric cancer, nasopharyngeal carcinoma, cervical cancer, vaginal cancer, vulvar cancer, squamous cell carcinoma of the head and neck, and Merkel cell carcinoma. (See https://clinicaltrials.gov/ct2/show/study/NCT02488759; see also https://clinicaltrials.gov/ct2/show/study/NCT0240886; https://clinicaltrials.gov/ct2/show/ NCT02426892)

In some embodiments, the present invention provides a method for treating a tumor in a patient in need thereof, comprising administering to the patient any of the compounds, salts or pharmaceutical compositions described herein. In some embodiments, the tumor comprises any of the cancers described herein. In some embodiments, the tumor comprises melanoma cancer. In some embodiments, the tumor comprises breast cancer. In some embodiments, the tumor comprises lung cancer. In some embodiments the tumor comprises small cell lung cancer (SCLC). In some embodiments the tumor comprises non-small cell lung cancer (NSCLC).

In some embodiments, the tumor is treated by arresting further growth of the tumor. In some embodiments, the tumor is treated by reducing the size (e.g., volume or mass) of the tumor by at least 5%, 10%, 25%, 50%, 75%, 90% or 99% relative to the size of the tumor prior to treatment. In some embodiments, tumors are treated by reducing the quantity of the tumors in the patient by at least 5%, 10%, 25%, 50%, 75%, 90% or 99% relative to the quantity of tumors prior to treatment.

The compounds and compositions, according to the method of the present invention, may be administered using any amount and any route of administration effective for treating or lessening the severity of a cancer, an autoimmune disorder, a primary immune deficiency, a proliferative disorder, an inflammatory disorder, a neurodegenerative or neurological disorder, schizophrenia, a bone-related disorder, liver disease, or a cardiac disorder. The exact amount required will vary from subject to subject, depending on the species, age, and general condition of the subject, the severity of the disease or condition, the particular agent, its mode of administration, and the like. Compounds of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compounds and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts.

Pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the disease or disorder being treated.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

### Treatment of cancer or dysplasia

Uses and methods are both considered, in the sense of the invention.

The invention further relates a method for treating or preventing cancer or dysplasia, comprising at least a step of administering to a patient in need thereof with an effective amount of a compound as defined above or anyone of its metabolites or pharmaceutically acceptable salt or anyone of their enantiomers or diastereoisomers, in particular 8-Chloro-N-(4-(trifluoromethoxy)phenyl)quinolin-2-amine.

The present invention is also related to the use of at least a compound as defined above or anyone of its metabolites or pharmaceutically acceptable salt or anyone of their enantiomers or diastereoisomers, and in particular 8-Chloro-N-(4-(trifluoromethoxy)phenyl)quinolin-2-amine under amorphous or crystalline form, or one of its metabolite or pharmaceutically acceptable salts thereof, in particular the N-glucuronide metabolite as defined above, according to the present invention for the manufacture of a pharmaceutical composition intended for the treatment of cancer or dysplasia.

The present invention further relates to a method of treatment of patients suffering from cancer or dysplasia, which comprises at least a step of administration to a patient suffering thereof of an effective amount of a compound as defined above or anyone of its metabolites or pharmaceutically acceptable salt or anyone of their enantiomers or diastereoisomers, and in particular 8-Chloro-N-(4-(trifluoromethoxy)phenyl)quinolin-2-amine under amorphous or crystalline form, or one of its metabolite or pharmaceutically acceptable salts thereof, in particular the N-glucuronide metabolite as defined above.

Human cancer cells, from different cancer sources are used to assess anti-tumoral activities of 8-Chloro-N-(4-(trifluoromethoxy)phenyl)quinolin-2-amine under any form or one of its pharmaceutically acceptable salt, as for example cell proliferation, viability and invasiveness potency. Cells are cultured in specific medium for each cancer cells in presence or not of 8-Chloro-N-(4-(trifluoromethoxy)phenyl)quinolin-2-amine under any form or one of its pharmaceutically acceptable salt during various durations, and for example 48 and 72 hours. At the end of culture, cell cytotoxicity, proliferation and viability can be evaluated with a luminescent or colorimetric test as CellTiter-Glo or MTT for example. Invasiveness abilities of cancer cells can be studied with transwell migration assay or with wound healing test.

### Doses and regimen

The invention further relates to a method for preventing and/or treating cancer or dysplasia, the method comprising the step of administering to a host an efficient amount of a compound as defined above, or a pharmaceutically acceptable salt thereof, in particular at various frequencies, in doses ranging from 10 to 1000 mg, in particular 25 to 800 mg, or even from 30 to 600 mg, and for example from 30 to 200 mg.

According to one embodiment, the treatment frequency may be once or twice a day, once every three days, once a week, once every 2 weeks or once every month.

According to a particular embodiment, the treatment is continuous or non continuous.

A "continuous treatment" means a long-term treatment which can be implemented with various administration frequencies, such as once every three days, or once a week, or once every two weeks or once every month.

The treatment period, i.e. when the treatment is non continuous, may vary between 1 week and three years, which includes 2 to 6 weeks, 3 months, 6 months, 1 year and three year.

According to one embodiment, a compound or anyone of its pharmaceutically acceptable salts as described herein, is administered at a dose varying from 25 to 300 mg, in particular varying from 25 to 200 mg, for example varying from 25 to 150 mg, and in particular varying from 25 to 100 mg. Doses ranging from 25 to 300 mg include doses of about 25, 50, 75, 100, 150, 200, 250 and 300 mg.

Said dosages may be adapted depending if the treatment is continuous or non continuous. It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of the quinoline compound of the present invention in the composition will also depend upon the particular compound in the composition.

All combinations of doses, frequencies and treatment period are encompassed within the scope of the present invention.

According to a particular embodiment, a compound according to the present invention, and more particularly 8-Chloro-N-(4-(trifluoromethoxy)phenyl)quinolin-2-amine or any pharmaceutically acceptable salt thereof, may be administered at various dosages and regimen and in particular once a day at doses ranging from 10 to 1000 mg, in particular 25 to 800 mg and more particularly from 25 to 400 mg as a continuous treatment or during a treatment period.

The treatment period may vary between one week and three years, which includes 2 to 6 weeks, 3 months, 6 months, 1 year and three year.

The quinoline derivative may be administered every day, once every three days, once a week, once every two weeks or once every month.

Several examples of doses and regimens are given herein below.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 25 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 50 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 75 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 100 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 150 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 300 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 400 mg once a day during the treatment period or as a continuous treatment.

More particularly the invention relates to a dosage and regimen where a compound according to the present invention or a pharmaceutically acceptable salt thereof, and more particularly 8-Chloro-N-(4-(trifluoromethoxy)phenyl)quinolin-2-amine or a pharmaceutically acceptable salt thereof, is administered at 600 mg once a day during the treatment period or as a continuous treatment.

More particularly the invention relates to a dosage and regimen where the N-glucuronide metabolite of formula (1) as defined above or a pharmaceutically acceptable salt thereof is administered at 50 mg once a day during the treatment period or as a continuous treatment.

More particularly the invention relates to a dosage and regimen where the N-glucuronide metabolite of formula (1) as defined above or a pharmaceutically acceptable salt thereof is administered at 150 mg once a day during the treatment period or as a continuous treatment.

More particularly the invention relates to a dosage and regimen where the N-glucuronide metabolite of formula (1) as defined above or a pharmaceutically acceptable salt thereof is administered at 300 mg every three days during the treatment period or as a continuous treatment.

More particularly the invention relates to a dosage and regimen where the N-glucuronide metabolite of formula (1) as defined above or a pharmaceutically acceptable salt thereof is administered at 500 mg once a day during the treatment period or as a continuous treatment.

More particularly the invention relates to a dosage and regimen where the N-glucuronide metabolite of formula (1) as defined above or a pharmaceutically acceptable salt thereof is administered at 700 mg once a day during the treatment period or as a continuous treatment.

More particularly the invention relates to a dosage and regimen where the N-glucuronide metabolite of formula (1) as defined above or a pharmaceutically acceptable salt thereof is administered at 1000 mg once a day during the treatment period or as a continuous treatment.

Thus, a compound according to the present invention may be implemented within pharmaceutical composition that may contain an effective amount of a compound as defined above or its metabolite under all the forms as described above, and one or more pharmaceutically acceptable excipients.

The composition may comprise a compound of this invention or a pharmaceutically acceptable salt thereof and a pharmaceutically acceptable excipient, carrier, adjuvant, or vehicle. In certain embodiments, a composition of this invention is formulated for administration to a patient in need of such composition.

The aforementioned excipients, carriers, adjuvants or vehicles are selected according to the dosage form and the desired mode of administration.

Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Said pharmaceutical form may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.The pharmaceutical form may in particularbe suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

More particularly, the route of administration may be oral or parenteral (IM, SC, IV) or intra-tumoral or be an *in-situ* administration.

Sustained release pharmaceutical compositions may be used.

For example, a compound or anyone of its or metabolites or pharmaceutically acceptable salt thereof as described herein can be administered by oral, parenteral, intravenous, transdermal, intramuscular, rectal, sublingual, mucosal, nasal, or other means. In addition, a compound or anyone of its or metabolites or pharmaceutically acceptable salt thereof as described herein can be administered in a form of pharmaceutical composition and/or unit dosage form.

In particular, pharmaceutical compositions of the invention may be administered orally and/or parenterally.

According to one exemplary embodiment, pharmaceutical compositions of the invention may be administered orally. Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, provided pharmaceutically acceptable compositions may be formulated in a suitable ointment containing the active component suspended or dissolved in one or more carriers. Carriers for topical administration of compounds of this invention include, but are not limited to, mineral oil, liquid petrolatum, white petrolatum, propylene glycol, polyoxyethylene, polyoxypropylene compound, emulsifying wax and water. Alternatively, provided pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, pharmaceutically acceptable compositions of this invention are formulated for oral administration. Such formulations may be administered with or without food. In some embodiments, pharmaceutically acceptable compositions of this invention are administered without food. In other embodiments, pharmaceutically acceptable compositions of this invention are administered with food.

Suitable dosage forms include, but are not limited to, capsules, tablets (including rapid dissolving and delayed release tablets), powder, syrups, oral suspensions and solutions for parenteral administration, and are more particularly capsules.

The pharmaceutical composition may also contain another drug for the treatment of cancer, well known to the man skilled in the art, in combination with a compound according to the present invention.

Advantageously, a compound or anyone of its or metabolites or pharmaceutically acceptable salt thereof, as described herein, may be administered in combination with one or more another anticancer drug and/or with radiotherapy.

Hence, the present invention further relates to a pharmaceutical combination of formula (I) or anyone of its metabolites or pharmaceutically acceptable salt or anyone of their enantiomers or diastereoisomers as defined above and of another anticancer drug.

When a compound or anyone of its or metabolites or pharmaceutically acceptable salt thereof as described herein, is administered in combination with another active ingredient, such as another anticancer drug, it may be administered under a unique dosage form.

Alternatively, such combination may be administered separately or simultaneously, for instance in a bolus. According to said particular embodiment, said combination can have the form of at least two pharmaceutical preparations. In other words, the combination can be in the form of a combination kit or product. The form of such combinations will depend on the mode of administration of each compound.

In such embodiment, the two pharmaceutical preparations may be administered sequentially (at different times) or concurrently (at the same time).

In other words, the administration of a compound of formula (I) or (I') or anyone of its or metabolites or pharmaceutically acceptable salt thereof and of said additional anti-tumoral therapy is simultaneous, separate or spread out over time.

For instance, according to some embodiments, each compound of the combination, or composition thereof, is administered by the same mode of administration (i.e.strictly oral). According to other embodiments, some compounds are administered by different modes of administration (i.e. oral and parenteral).

The combination can be administered repeatedly over the course of several cycles according to a protocol which depends on the nature and on the stage of the prostate cancer to be treated and also on the patient to be treated (age, weight, previous treatment(s), etc.). The protocol can be determined by any practitioner specializing in oncology.

According to a particular embodiment of the invention, radiotherapy treatments may also be administered simultaneously or sequentially.

Among other anticancer drug, the following may be cited:
- Androgen receptor inhibitors, such as enzalutamide (Xtandi®, Astellas/Medivation), abiraterone (Zytiga®, Centocor/Ortho), antagonist of gonadotropin-releasing hormone (GnRH) receptor such as degaralix, Firmagon®, Ferring Pharmaceuticals)
- **Antiapoptotics,** such as venetoclax (Venclexta®, AbbVie/Genentech), blinatumomab (Blincyto®, Amgen), navitoclax (ABT-263, Abbott);
- **Antiproliferative and Antimitotic agents,** such as vinca alkaloids (which include vinblastine, vincristine);
- **Antibiotics** such as dactinomycin, daunorubicin, doxorubicin, idarubicin, anthracyclines, mitoxantrone, bleomycins, plicamycin (mithramycin), and mitomycin;
- **L-asparaginase;**
- **Antiplatelet agents;**
- **Antiproliferative/antimitotic alkylating agents** such as nitrogen mustards cyclophosphamide and analogs (which include melphalan, chlorambucil, hexamethylmelamine, and thiotepa), alkyl nitrosoureas (which include carmustine) and analogs, streptozocin, and triazenes (which include dacarbazine);
- **Antiproliferative/antimitotic antimetabolites** such as folic acid analogs (which include methotrexate), aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (Temodal®); kinesin spindle protein inhibitors, such as SB715992 or SB743921 from GlaxoSmithKline, or pentamidine/chlorpromazine from CombinatoRx; MEK inhibitors such as ARRY142886 from Array BioPharma, AZd₆244 from AstraZeneca, PD181461 from Pfizer and leucovorin;
- **Antimigratory agents;**
- **Angiogenesis inhibitors,** such as TNP-470;
- **Aromatase inhibitors,** such as letrozole and anastrozole, exemestane;
- **Angiotensin**
- **Anti-sense oligonucleotides,** such as antisense nucleic acids directed toward miR-124;
- **Anticoagulants,** such as heparin, synthetic heparin salts, and other inhibitors of thrombin;
- **Arginine inhibitors,** such as AEB1102 (pegylated recombinant arginase, Aeglea Biotherapeutics) and CB-1158 (Calithera Biosciences);
- **Bone resorption inhibitors,** such as Denosumab (Xgeva®, Amgen), bisphosphonates such as zoledronic acid (Zometa®, Novartis);
- **CC chemokine receptor 4 (CCR4) inhibitors,** such as mogamulizumab (Poteligeo®, Kyowa Hakko Kirin, Japan);
- **CDK inhibitors,** such as CDK4/CDK6 inhibitors, such as palbociclib (Ibrance®, Pfizer); ribociclib (Kisqali®, Novartis); abemaciclib (Ly2835219, Eli Lilly); and trilaciclib (G1T28, G1 Therapeutics);
- **Cell cycle inhibitors and differentiation inducers,** such as as tretinoin;
- **Corticosteroids,** such as cortisone, dexamethasone, hydrocortisone, methylprednisolone, prednisone, and prednisolone;
- **DNA damaging agents** such as actinomycin, amsacrine, busulfan, carboplatin, chlorambucil, cisplatin, cyclophosphamide (CYTOXAN®), dactinomycin, daunorubicin, doxorubicin, epirubicin, iphosphamide, melphalan, merchlorethamine, mitomycin, mitoxantrone, nitrosourea, procarbazine, taxol, taxotere, teniposide, etoposide, and triethylenethiophosphoramide;
- **Fibrinolytic agents,** such as tissue plasminogen activator, streptokinase, urokinase, aspirin, dipyridamole, ticlopidine, and clopidogrel;
- **Folate antagonists;**
- **FLT3 receptor inhibitors,** such as enzalutamide, abiraterone, apalutamide, erlotinib, crizotinib, niraparib,olaparib, osimertinib, regorafenib, sunitinib, lestaurtinib, midostaurin, gilteritinib, semaxinib, linifanib, fostamatinib, pexidartinib, sorafenib, cabozantinib, ponatinib, ilorasertib, pacritinib, famitinib, pexidartinib, quizartinib;
- **Glutaminase inhibitors,** such as CD-839 (Calithera Biosciences);
- **Growth Factor Signal transduction kinase inhibitors;**
- **Growth factor Inhibitors,** such as vascular endothelial growth factor inhibitors and fibroblast growth factor inhibitors, such as olaratumab (Lartruvo®; Eli Lilly), cetuximab (Erbitux®, Eli Lilly); necitumumab (Portrazza®, Eli Lilly), panitumumab (Vectibix®, Amgen); and osimertinib (targeting activated EGFR, Tagrisso®, AstraZeneca);
- **Hedgehog pathway inhibitors,** such as sonidegib (Odomzo®, Sun Pharmaceuticals); and vismodegib (Erivedge®, Genentech);
- **Histone deacetylase (HDAC) inhibitors,** such as vorinostat (Zolinza®, Merck); romidepsin (Istodax®, Celgene); panobinostat (Farydak®, Novartis); belinostat (Beleodaq®, Spectrum Pharmaceuticals); entinostat (SNDX-275, Syndax Pharmaceuticals) (NCT00866333); and chidamide (Epidaza®, HBI-8000, Chipscreen Biosciences, China);
- **Hormones and analogs thereof,** such as estrogen, tamoxifen, goserelin, bicalutamide, and nilutamide);
- **Isocitrate dehydrogenase (IDH) inhibitors,** such as AG120 (Celgene; NCT02677922); AG221 (Celgene, NCT02677922; NCT02577406); BAY1436032 (Bayer, NCT02746081); IDH305 (Novartis, NCT02987010)
- **Isoflavones** such as genistein;
- **Immunosuppressives,** such as tacrolimus, sirolimus, azathioprine, and mycophenolate;
- **Inhibitors of p53 suppressor proteins,** such as ALRN-6924 (Aileron);
- **Inhibitors of transforming growth factor-beta (TGF-beta or TGFβ),** such as NIS793 (Novartis), fresolimumab (GC1008; Sanofi-Genzyme), M7824 (Merck KgaA - formerly MSB0011459X);
- **iNKT cell agonists** such as ABX196 (Abivax);
- **mTOR inhibitors,** such as everolimus (Afinitor®, Novartis); temsirolimus (Torisel®, Pfizer); and sirolimus (Rapamune®, Pfizer);
- **Microtubule-inhibiting drugs,** such as taxanes (which include paclitaxel, docetaxel), vinblastin, nocodazole, epothilones, vinorelbine) (NAVELBINE®), and epipodophyllotoxins (etoposide, teniposide);
- **Nitric oxide donors;**
- **Nucleoside inhibitors,** such as trabectedin (guanidine alkylating agent, Yondelis®, Janssen Oncology), mechlorethamine (alkylating agent, Valchlor®, Aktelion Pharmaceuticals); vincristine (Oncovin®, Eli Lilly; Vincasar®, Teva Pharmaceuticals; Marqibo®, Talon Therapeutics); temozolomide (prodrug to alkylating agent 5-(3-methyltriazen-1-yl)-imidazole-4-carboxamide (MTIC) Temodar®, Merck); cytarabine injection (ara-C, antimetabolic cytidine analog, Pfizer); lomustine (alkylating agent, CeeNU®, Bristol-Myers Squibb; Gleostine®, NextSource Biotechnology); azacitidine (pyrimidine nucleoside analog of cytidine, Vidaza®, Celgene); omacetaxine mepesuccinate (cephalotaxine ester) (protein synthesis inhibitor, Synribo®; Teva Pharmaceuticals); asparaginase *Erwinia chrysanthemi* (enzyme for depletion of asparagine, Elspar®, Lundbeck; Erwinaze®, EUSA Pharma); eribulin mesylate (microtubule inhibitor, tubulin-based antimitotic, Halaven®, Eisai); cabazitaxel (microtubule inhibitor, tubulin-based antimitotic, Jevtana®, Sanofi-Aventis); capacetrine (thymidylate synthase inhibitor, Xeloda®, Genentech); bendamustine (bifunctional mechlorethamine derivative, believed to form interstrand DNA cross-links, Treanda®, Cephalon/Teva); ixabepilone (semi-synthetic analog of epothilone B, microtubule inhibitor, tubulin-based antimitotic, Ixempra®, Bristol-Myers Squibb); nelarabine (prodrug of deoxyguanosine analog, nucleoside metabolic inhibitor, Arranon®, Novartis); clorafabine (prodrug of ribonucleotide reductase inhibitor, competitive inhibitor of deoxycytidine, Clolar®, Sanofi-Aventis); and trifluridine and tipiracil (thymidine-based nucleoside analog and thymidine phosphorylase inhibitor, Lonsurf®, Taiho Oncology);
- **PI3K inhibitors,** such as idelalisib (Zydelig®, Gilead), alpelisib (BYL719, Novartis), taselisib (GDC-0032, Genentech/Roche); pictilisib (GDC-0941, Genentech/Roche); copanlisib (BAY806946, Bayer); duvelisib (formerly IPI-145, Infinity Pharmaceuticals); PQR309 (Piqur Therapeutics, Switzerland); and TGR1202 (formerly RP5230, TG Therapeutics);
- **Platinum coordination complexes** (such as cisplatin, oxiloplatin, carboplatin, nedaplatin, picoplatin, procarbazine, mitotane, satraplatin and aminoglutethimide;
- **Poly ADB ribose polymerase (PARP) inhibitor,** such as those selected from: olaparib (Lynparza®, AstraZeneca); rucaparib (Rubraca®, Clovis Oncology); niraparib (Zejula®, Tesaro); talazoparib (MDV3800/BMN 673/LT00673, Medivation/Pfizer/Biomarin); veliparib (ABT-888, AbbVie); and BGB-290 (BeiGene, Inc.) ;
- **Proteasome inhibitors,** such as everolimus (Afinitor®, Novartis); temsirolimus (Torisel®, Pfizer); and sirolimus (Rapamune®, Pfizer), bortezomib (Velcade®, Takeda); carfilzomib (Kyprolis®, Amgen); and ixazomib (Ninlaro®, Takeda);
- **Pyrimidine & Purine analogs,** such as floxuridine, capecitabine, and cytarabine;
- **Receptor blockers, Antisecretory agents,** such as breveldin;
- **Selective estrogen receptor modulator (SERM),** such as raloxifene (Evista®, Eli Lilly);
- **Therapeutic antibodies,** such as those selected from: anti-TNF antibodies, anti-VEGF antibodies, anti-EGFR antibodies, anti-PD-1 antibodies, anti-HER2 antibodies, anti-CD20 antibodies, anti-IL17 antibodies, and anti-CTLA4 antibodies, anti-PDLl, anti-CD25, anti-α4integrin, anti-IL6R, anti-C5, anti-ILl, anti-TPO, anti-IL12/23, anti-EPCAM/CD3, anti-CD30, anti-CD80/86, anti-anthrax, anti-CCR4, anti-CD6, anti-CD19, anti-α4β7, anti-IL6, anti-VEGFR-2, anti-SLAMF7, anti-GD2, anti-IL17A, anti-PCSK9, anti-IL5, anti-CD22, anti-IL4, anti-PDGFRa, anti-IL17RA and anti-TcdB, and such as those selected from: Abagovomab, Abatacept, Abciximab, Abituzumab, Abrilumab, Actoxumab, Adalimumab, Adecatumab, Aducanumab, Aflibercept, Afutuzymab, Alacizumab, Alefacept, Alemtuzumab, Alirocumab, Altumomab, Amatixumab, Anatumomab, Anetumab, Anifromumab, Anrukinzumab, Apolizumab, Arcitumomab, Ascrinvacumab, Aselizumab, Atezolizumab, Atinumab, Altizumab, Atorolimumab, Bapineuzumab, Basiliximab, Bavituximab, Bectumomab, Begelomab, Belatacept, Belimumab, Benralizumab, Bertilimumab, Besilesomab, Bevacizumab, Bezlotoxumab, Biciromab, Bimagrumab, Bimekizumab, Bivatuzumab, Blinatumomab, Blosozumab, Bococizumab, Brentuximab, Briakimumab, Brodalumab, Brolucizumab, Bronticizumab, Canakinumab, Cantuzumab, Caplacizumab, Capromab, Carlumab, Catumaxomab, Cedelizumab, Certolizumab, Cetixumab, Citatuzumab, Cixutumumab, Clazakizumab, Clenoliximab, Clivatuzumab, Codrituzumab, Coltuximab, Conatumumab, Concizumab, Crenezumab, Dacetuzumab, Daclizumab, Dalotuzumab, Dapirolizumab, Daratumumab, Dectrekumab, Demcizumab, Denintuzumab, Denosumab, Derlotixumab, Detumomab, Dinutuximab, Diridavumab, Dorlinomab, Drozitumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Enavatuzumab, Enfortumab, Enlimomab, Enoblituzumab, Enokizumab, Enoticumab, Ensituximab, Epitumomab, Epratuzomab, Erlizumab, Ertumaxomab, Etanercept, Etaracizumab, Etrolizumab, Evinacumab, Evolocumab, Exbivirumab, Fanolesomab, Faralimomab, Farletuzomab, Fasimumab, Felvizumab, Fezkimumab, Ficlatuzumab, Figitumumab, Firivumab, Flanvotumab, Fletikumab,Fontolizumab, Foralumab, Foravirumab, Fresolimumab, Fulramumab, Futuximab, Galiximab, Ganitumab, Gantenerumab, Gavilimomab, Gemtuzumab, Gevokizumab, Girentuximab, Glembatumumab, Golimumab, Gomiliximab, Guselkumab, Ibalizumab, Ibritumomab, Icrucumab, Idarucizumab, Igovomab, Imalumab, Imciromab, Imgatuzumab, Inclacumab, Indatuximab, Indusatumab, Infliximab, Intetumumab, Inolimomab, Inotuzumab, Ipilimumab, Iratumumab, Isatuximab, Itolizumab, Ixekizumab, Keliximab, Labetuzumab, Lambrolizumab, Lampalizumab, Lebrikizumab, Lemalesomab, Lenzilumab, Lerdelimumab, Lexatumumab, Libivirumab, Lifastuzumab, Ligelizumab, Lilotomab, Lintuzumab, Lirilumab, Lodelcizumab, Lokivetmab, Lorvotuzumab, Lucatumumab, Lulizumab, Lumiliximab, Lumretuzumab, Mapatumumab, Margetuximab, Maslimomab, Mavrilimumab, Matuzumab, Mepolizumab, Metelimumab, Milatuzumab, Minetumomab, Mirvetuximab, Mitumomab, Mogamulizumab, Morolimumab, Motavizumab, Moxetumomab, Muromonab-CD3, Nacolomab, Namilumab, Naptumomab, Namatumab, Natalizumab, Nebacumab, Necitumumab, Nemolizumab, Nerelimomab, Nesvacumab, Nimotuzumab, Nivolumab, Nofetumomab, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab, Oregovomab, Orticumab, Otelixizumab, Oltertuzumab, Oxelumab, Ozanezumab, Ozoralizumab, Pagibaximab, Palivizumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab, Pintumomab, Polatuzumab, Ponezumab, Priliximab, Pritumumab, Quilizumab, Racotumomab, Radretumab, Rafivirumab, Ralpancizumab, Ramucirumab, Ranibizumab, Raxibacumab, Refanezumab, Regavirumab, Reslizumab, Rilonacept, Rilotumumab, Rinucumab, Rituximab, Robatumumab, Roledumab, Romosozumab, Rontalizumab, Rovelizumab, Ruplizumab, Sacituzumab, Samalizumab, Sarilumab, Satumomab, Secukimumab, Seribantumab, Setoxaximab, Sevirumab, Sibrotuzumab, Sifalimumab, Siltuximab, Siplizumab, Sirukumab, Sofituzumab, Solanezumab, Solitomab, Sonepcizumab, Sontuzumab, Stamulumab, Sulesomab, Suvizumab, Tabalumab, Tacatuzumab, Tadocizumab, Talizumab, Tanezumab, Taplitumomab, Tarextumab, Tefibazumab, Telimomab aritox, Tenatumomab, Teneliximab, Teplizumab, Tesidolumab, TGN 1412, Ticlimumab, Tildrakizumab, Tigatuzumab, TNX-650, Tocilizumab, Toralizumab, Tosatoxumab, Tositumomab, Tovetumab, Tralokimumab, Trastuzumab, TRBS07, Tregalizumab, Tremelimumab, Trevogrumab, Tucotuzumab, Tuvirumab, Ublituximab, Ulocuplumab, Urelumab, Urtoxazumab, Ustekimumab, Vandortuzumab, Vantictumab, Vanucizumab, Vapaliximab, Varlimumab, Vatelizumab, Vedolizumab, Veltuzumab, Vepalimomab, Vesencumab, Visilizumab, Volocixumab, Vorsetuzumab, Votumumab, Zalutumimab, Zanolimumab, Zatuximab, Ziralimumab, Ziv-Aflibercept, and Zolimomab;
- **Topoisomerase inhibitors,** such as doxorubicin, daunorubicin, dactinomycin, eniposide, epirubicin, etoposide, idarubicin, irinotecan, mitoxantrone, topotecan, and irinotecan;
- **Toxins,** such as Cholera toxin, ricin, *Pseudomonas* exotoxin, *Bordetella pertussis* adenylate cyclase toxin, diphtheria toxin, and caspase activators;

**Kinase or VEGF inhibitors,** such as regorafenib (Stivarga®, Bayer); vandetanib (Caprelsa®, AstraZeneca); axitinib (Inlyta®, Pfizer); and lenvatinib (Lenvima®, Eisai); Raf inhibitors, such as sorafenib (Nexavar®, Bayer AG and Onyx); dabrafenib (Tafinlar®, Novartis); and vemurafenib (Zelboraf®, Genentech/Roche); MEK inhibitors, such as cobimetanib (Cotellic®, Exelexis/Genentech/Roche); trametinib (Mekinist®, Novartis); Bcr-Abl tyrosine kinase inhibitors, such as imatinib (Gleevec®, Novartis); nilotinib (Tasigna®, Novartis); dasatinib (Sprycel®, BristolMyersSquibb); bosutinib (Bosulif®, Pfizer); and ponatinib (Inclusig®, Ariad Pharmaceuticals); Her2 and EGFR inhibitors, such as gefitinib (Iressa®, AstraZeneca); erlotinib (Tarceeva®, Genentech/Roche/Astellas); lapatinib (Tykerb®, Novartis); afatinib (Gilotrif®, Boehringer Ingelheim); osimertinib (targeting activated EGFR, Tagrisso®, AstraZeneca); and brigatinib (Alunbrig®, Ariad Pharmaceuticals); c-Met and VEGFR2 inhibitors, such as cabozanitib (Cometriq®, Exelexis); and multikinase inhibitors, such as sunitinib (Sutent®, Pfizer); pazopanib (Votrient®, Novartis); ALK inhibitors, such as crizotinib (Xalkori®, Pfizer); ceritinib (Zykadia®, Novartis); and alectinib (Alecenza®, Genentech/Roche); Bruton's tyrosine kinase inhibitors, such as ibrutinib (Imbruvica®, Pharmacyclics/Janssen); and Flt3 receptor inhibitors, such as midostaurin (Rydapt®, Novartis), tivozanib (Aveo Pharmaecuticals); vatalanib (Bayer/Novartis); lucitanib (Clovis Oncology); dovitinib (TKI258, Novartis); Chiauanib (Chipscreen Biosciences); CEP-11981 (Cephalon); linifanib (Abbott Laboratories); neratinib (HKI-272, Puma Biotechnology); radotinib (Supect®, IY5511, Il-Yang Pharmaceuticals, S. Korea); ruxolitinib (Jakafi®, Incyte Corporation); PTC299 (PTC Therapeutics); CP-547,632 (Pfizer); foretinib (Exelexis, GlaxoSmithKline); quizartinib (Daiichi Sankyo) and motesanib (Amgen/Takeda);

In a non-limitative manner, compounds of the invention may be combined, alone or in the form of a kit-of-parts, to one or more of the following anti-cancer drugs or compounds: ABVD, AC, ACE, Abiraterone (Zytiga®), Abraxane, Abstral, Actinomycin D, Actiq, Adriamycin, Afatinib (Giotrif®), Afinitor, Aflibercept (Zaltrap®), Aldara, Aldesleukin (IL-2, Proleukin or interleukin 2), Alemtuzumab (MabCampath), Alkeran, Amsacrine (Amsidine, m-AMSA), Amsidine, Anastrozole (Arimidex®), Ara C, Aredia, Arimidex, Aromasin, Arsenic trioxide (Trisenox®, ATO), Asparaginase (Crisantaspase®, Erwinase®), Axitinib (Inlyta®), Azacitidine (Vidaza®), BEACOPP, BEAM, Bendamustine (Levact®), Bevacizumab (Avastin), Bexarotene (Targretin®), Bicalutamide (Casodex®), Bleomycin, Bleomycin, etoposide and platinum (BEP), Bortezomib (Velcade®), Bosulif, Bosutinib (Bosulif), Brentuximab (Adcetris®), Brufen, Buserelin (Suprefact®), Busilvex, Busulfan (Myleran, Busilvex), CAPE-OX, CAPOX, CAV, CAVE, CCNU, CHOP, CMF, CMV, CVP, Cabazitaxel (Jevtana®), Cabozantinib (Cometriq®), Caelyx, Calpol, Campto, Capecitabine (Xeloda®), Caprelsa, Carbo MV, CarboTaxol, Carboplatin, Carboplatin and etoposide, Carboplatin and paclitaxel, Carmustine (BCNU, Gliadel®), Casodex, Ceritinib (Zykadia®), Cerubidin, Cetuximab (Erbitux®), ChlVPP, Chlorambucil (Leukeran®), Cisplatin, Cisplatin and Teysuno, Cisplatin and capecitabine (CX), Cisplatin, etoposide and ifosfamide (PEI), Cisplatin, fluorouracil (5-FU) and trastuzumab, Cladribine (Leustat®, LITAK), Clasteon, Clofarabine (Evoltra®), Co-codamol (Kapake®, Solpadol®, Tylex®), Cometriq, Cosmegen, Crisantaspase, Crizotinib (Xalkori®), Cyclophosphamide, Cyclophosphamide, thalidomide and dexamethasone (CTD), Cyprostat,Cyproterone acetate (Cyprostat®), Cytarabine (Ara C, cytosine arabinoside), Cytarabine into spinal fluid, Cytosine arabinoside, DHAP,DTIC, Dabrafenib (Tafinlar®),Dacarbazine (DTIC), Dacogen, Dactinomycin (actinomycin D, Cosmegen®), Dasatinib (Sprycel), Daunorubicin, De Gramont, Decapeptyl SR, Decitabine (Dacogen®), Degarelix (Firmagon®), Denosumab (Prolia®, Xgeva®), Depocyte, Dexamethasone, Diamorphine, Disodium pamidronate, Disprol, Docetaxel (Taxotere®), Docetaxel, cisplatin and fluorouracil (TPF), Doxifos, Doxil, Doxorubicin (Adriamycin), Doxorubicin and ifosfamide (Doxifos), Drogenil, Durogesic, EC, ECF, EOF, EOX, EP, ESHAP, Effentora, Efudix, Eldisine, Eloxatin, Enzalutamide, Epirubicin (Pharmorubicin®), Epirubicin cisplatin and capecitabine (ECX), Epirubicin, carboplatin and capecitabine (ECarboX), Eposin, Erbitux, Eribulin (Halaven®), Erlotinib (Tarceva®), Erwinase, Estracyt, Etopophos, Etoposide (Eposin®, Etopophos®, Vepesid®), Everolimus (Afinitor®), Evoltra, Exemestane (Aromasin®), FAD, FEC, FEC-T chemotherapy, FMD, FOLFIRINOX, FOLFOX, Faslodex, Femara, Fentanyl, Firmagon, Fludara, Fludarabine (Fludara®), Fludarabine, cyclophosphamide and rituximab (FCR), Fluorouracil (5FU), Flutamide, Folinic acid, fluorouracil and irinotecan (FOLFIRI), Fulvestrant (faslodex®), G-CSF, Gefitinib (Iressa), GemCarbo (gemcitabine and carboplatin), GemTaxol, Gemcitabine (Gemzar), Gemcitabine and capecitabine (GemCap), Gemcitabine and cisplatin (GC), Gemcitabine and paclitaxel (GemTaxol®),Gemzar,Giotrif, Gliadel, Glivec, Gonapeptyl, Depot, Goserelin (Zoladex®), Goserelin (Zoladex®, Novgos®), Granulocyte colony stimulating factor (G-CSF), Halaven,Herceptin, Hycamtin, Hydrea, Hydroxycarbamide (Hydrea®), Hydroxyurea, I-DEX, ICE, IL-2, IPE, Ibandronic acid, Ibritumomab (Zevalin®), Ibrutinib (Imbruvica®), Ibuprofen (Brufen®, Nurofen®), Iclusig, Idarubicin (Zavedos®), Idarubicin and dexamethasone, Idelalisib (Zydelig®), Ifosfamide (Mitoxana®), Imatinib (Glivec®), Imiquimod cream (Aldara®), Imnovid, Instanyl, Interferon (Intron A), Interleukin, Intron A, Ipilimumab (Yervoy®), Iressa, Irinotecan (Campto®), Irinotecan and capecitabine (Xeliri®), Irinotecan de Gramont, Irinotecan modified de Gramont, Javlor, Jevtana, Kadcyla, Kapake, Keytruda, Lanreotide (Somatuline®), Lanvis, Lapatinib (Tyverb®), Lenalidomide (Revlimid®), Letrozole (Femara®), Leukeran, Leuprorelin (Prostap®, Lutrate®), Leustat, Levact, Liposomal doxorubicin, Litak, Lomustine (CCNU), Lynparza, Lysodren, MIC, MMM, MPT, MST Continus, MVAC, MVP, MabCampath, Mabthera, Maxtrex, Medroxyprogesterone acetate (Provera), Megace, Megestrol acetate (Megace®), Melphalan (Alkeran®), Mepact, Mercaptopurine (Xaluprine®), Methotrexate (Maxtrex), Methyl prednisolone, Mifamurtide (Mepact®), Mitomycin C, Mitotane, Mitoxana, Mitoxantrone (Mitozantrone®), Morphgesic SR, Morphine, Myleran, Myocet, Nab-paclitaxel, Nab-paclitaxel (Abraxane®), Navelbine, Nelarabine (Atriance®), Nexavar, Nilotinib (Tasigna®), Nintedanib (Vargatef®), Nipent, Nivolumab (Opdivo®), Novgos, Nurofen, Obinutuzumab (Gazyvaro®), Octreotide, Ofatumumab (Arzerra®), Olaparib (Lynparza®), Oncovin, Onkotrone, Opdivo, Oramorph, Oxaliplatin (Eloxatin), Oxaliplatin and capecitabine (Xelox®), PAD, PC (paclitaxel and carboplatin, CarboTaxol), PCV, PE, PMitCEBO, POMB/ACE, Paclitaxel (Taxol®), Paclitaxel and carboplatin, Pamidronate, Panadol, Panitumumab (Vectibix®), Paracetamol, Pazopanib (Votrient®), Pembrolizumab (Keytruda), Pemetrexed (Alimta®), Pemetrexed and carboplatin, Pemetrexed and cisplatin, Pentostatin (Nipent®), Perjeta, Pertuzumab (Perjeta®), Pixantrone (Pixuvri®), Pixuvri, Pomalidomide (Imnovid®), Ponatinib, Potactasol, Prednisolone, Procarbazine, Proleukin, Prolia, Prostap, Provera, Purinethol, R-CHOP, R-CVP, R-DHAP, R-ESHAP, R-GCVP, RICE, Raloxifene, Raltitrexed (Tomudex®), Regorafenib (Stivarga®), Revlimid, Rituximab, (Mabthera®), Sevredol, Sodium clodronate (Bonefos®, Clasteon®, Loron®), Solpadol, Sorafenib (Nexavar®), Steroids (dexamethasone, prednisolone, methylprednisolone), Streptozocin (Zanosar®), Sunitinib (Sutent®), Sutent, TAC, TIP, Tafinlar, Tamoxifen, Tarceva, Targretin, Tasigna, Taxol, Taxotere, Taxotere and cyclophosphamide (TC), Temodal, Temozolomide, (Temodal®), Temsirolimus (Torisel®), Tepadina, Teysuno, Thalidomide, Thiotepa (Tepadina®), Tioguanine (thioguanine®, 6-TG, 6-tioguanine), Tomudex, Topotecan (Hycamtin, Potactasol), Torisel, Trabectedin (Yondelis), Trastuzumab (Herceptin®), Trastuzumab emtansine (Kadcyla®), Treosulfan, Tretinoin (Vesanoid®, ATRA), Triptorelin (Decapeptyl SR®, Gonapeptyl Depot®), Trisenox, Tylex, Tyverb, VIDE, Vandetanib (Caprelsa®), Vargatef, VelP, Vectibix, Velbe, Velcade, Vemurafenib (Zelboraf®), Vepesid, Vesanoid, Vidaza, Vinblastine (Velbe®), Vincristine, Vincristine, actinomycin D (dactinomycin®) and cyclophosphamide (VAC), Vincristine, actinomycin and ifosfamide (VAI), Vincristine, doxorubicin and dexamethasone (VAD), Vindesine (Eldisine®), Vinflunine (Javlor®), Vinorelbine (Navelbine®),Vismodegib (Erivedge®), Votrient, XELOX, Xalkori, Xeloda, Xgeva, Xtandi, Yervoy, Yondelis, Z-DEX, Zaltrap, Zanosar, Zavedos, Zelboraf, Zevalin, Zoladex (breast cancer), Zoladex (prostate cancer), Zoledronic acid (Zometa®), Zometa, Zomorph, Zydelig, Zytiga.

According to a particular embodiment, a compound or anyone of its or metabolites or pharmaceutically acceptable salt thereof, as described herein, can be combined with various chemotherapies, immunotherapy (e.g. check-point inhibitors, monoclonal antibodies), anti-tumoral vaccines, RNA vaccines, magnetic particles, intravascular microrobots, radiotherapy, surgery, ultrasounds or other anti-tumoral therapies.

Therefore, the present invention further provides a compound of formula (I) or (I') or anyone of its or metabolites or pharmaceutically acceptable salt thereof for use as an antitumor agent intended for patients who are also treated with anyone of immunotherapy, anti-tumoral vaccines, RNA vaccines, radiotherapy, surgery, ultrasounds or other anti-tumoral therapies.

Where a prevention and/or treatment method is provided, the present invention also provides the corresponiding use for preventing and/or treating cancer or dysplasia. Likewise, where a use for preventing and/or treating cancer or dysplasia is provided, the present invention also provides the corresponiding prevention and/or treatment method. In some embodiments, the present invention provides a method for preventing and/or treating cancer or dysplasia as described herein, comprising administering a compound or a pharmaceutically acceptable salt thereof, as described herein. In some embodiments, the present invention provides a method for preventing and/or treating cancer or dysplasia as described herein, comprising measuring a presence and/or expression level of miR-124 as described herein.

Herein is further provided a quinoline derivative of formula (I') or anyone of its metabolites or pharmaceutically acceptable salt or anyone of their enantiomers or diastereoisomers for use according to the present invention, wherein the use is intended for a patient whose level of a compound of formula (I'), or a pharmaceutically acceptable salt thereof, in a blood, plasma, tissue, saliva, and/or serum sample of the patient is measured during the use.

Herein is further provided a quinoline derivative of formula (I') or anyone of its metabolites or pharmaceutically acceptable salt or anyone of their enantiomers or diastereoisomers for use according to the present invention, wherein the use is intended for a patient whose level of a compound of formula (IVa), or a pharmaceutically acceptable salt thereof, in a blood, plasma, tissue, saliva, and/or serum sample of the patient is measured during the use.

Herein is further provided a quinoline derivative of formula (I') or anyone of its metabolites or pharmaceutically acceptable salt or anyone of their enantiomers or diastereoisomers for use according to the present invention, wherein the use is intended for a patient whose level of a compound of formulas (I') and (IVa), or their pharmaceutically acceptable salt thereof, in a blood, plasma, tissue, saliva, and/or serum sample of the patient is measured during the use.

### Further Exemplary Embodiments

1. A compound of Formula (I): 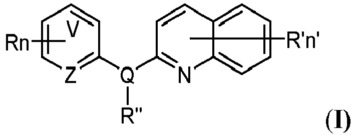 or a pharmaceutically acceptable salt thereof, wherein:
   Z is C or N;
   V is C or N; 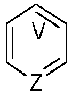 means an aromatic ring wherein V is C or N, and when V is N, V is ortho, meta or para relative to Z;
   each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R₁, -NR₁-C(=O)-R₁, -NR₁-C(=O)-NR₁R₂, -SO₂-NR₁R₂, -SO₃H, -O-SO₂-OR₃, -O-P(=O)-(OR₃)(OR₄), -O-CH₂-COOR₃, (C₁-C₃)alkyl, said alkyl being optionally mono- or di-substituted by a hydroxyl group, a group of formula (IIa): 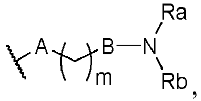 or a group of formula (IIIa): 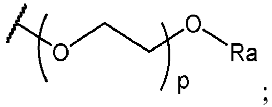
   Q is N or O, provided that R" does not exist when Q is O;
   each of R₁ and R₂ is independently hydrogen or (C₁-C₃)alkyl;
   each of R₃ and R₄ is independently hydrogen, Li⁺, Na⁺, K⁺, N⁺(Ra)₄ or benzyl;
   n is 1, 2 or 3;
   n' is 1, 2 or 3;
   each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy, -OP(=O)-(OR₃)(OR₄), -CN, a group of formula (IIa): 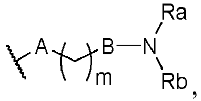 or a group of formula (IIIa): 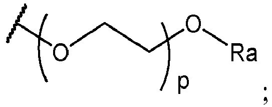
   A is a covalent bond, oxygen, or NH;
   B is a covalent bond or NH;
   m is 1, 2, 3, 4 or 5;
   p is 1, 2 or 3;
   each of Ra and Rb is independently hydrogen, (C₁-C₅)alkyl, or (C₃-C₆)cycloalkyl, or
   Ra and Rb form together with the nitrogen atom to which they are attached a saturated 5- or 6-membered heterocycle, said heterocycle being optionally substituted by one or more Ra, provided that when R' is a group (IIa) or (IIa), n' may be 2 or 3 only if other R' groups are different from said group (IIa) or (IIa); and
   R" is hydrogen, (C₁-C₄)alkyl, or a group of formula (IIa) as defined herein,
   for use for treating or preventing cancer or dysplasia.
2. The compound of embodiment 1, wherein the compound is of formula (**Ia**): 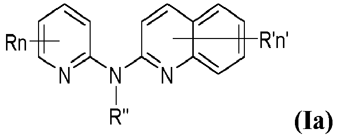 or a pharmaceutically acceptable salt thereof.
3. The compound of embodiment 1, wherein the compound is of formula (**Ib**): 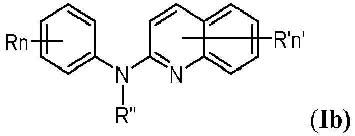 or a pharmaceutically acceptable salt thereof.
4. The compound of embodiment 1, wherein the compound is of formula **(Ic):** 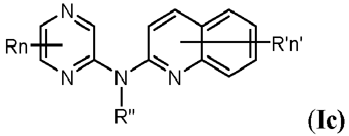 or a pharmaceutically acceptable salt thereof.
5. The compound of embodiment 1, wherein the compound is of formula **(Ib'):** 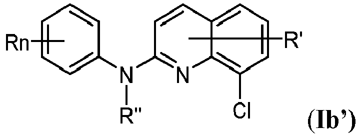 or a pharmaceutically acceptable salt thereof.
6. The compound of embodiment 1, wherein the compound is of formula **(Id):** 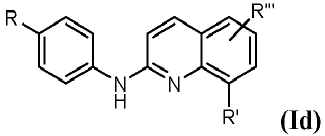 or a pharmaceutically acceptable salt thereof.
7. A compound of formula **(IV):** 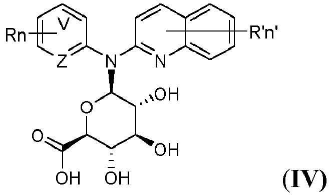 or a pharmaceutically acceptable salt thereof, wherein each of variables V, Z, R, R', n, and n' is as described in embodiment 1, for use for treating or preventing cancer or dysplasia.
8. The compound of embodiment 7, wherein the compound is of formula **(IVa):** 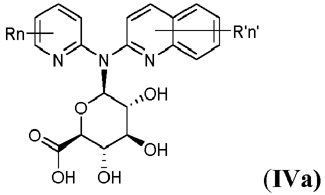 or a pharmaceutically acceptable salt thereof.
9. The compound of embodiment 7, wherein the compound is of formula **(IVb):** 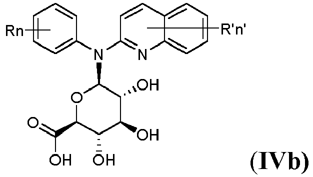 or a pharmaceutically acceptable salt thereof.
10. The compound of embodiment 7, wherein the compound is of formula **(IVc):** 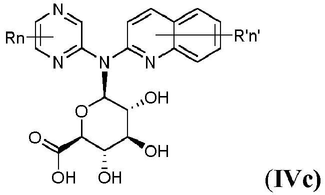 or a pharmaceutically acceptable salt thereof.
11. The compound of embodiment 7, wherein the compound is of formula **(IVb'):** 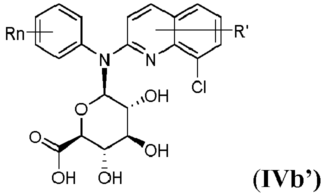 or a pharmaceutically acceptable salt thereof.
12. The compound of embodiment 7, wherein the compound is of formula **(IVd):** 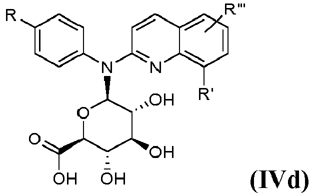 or a pharmaceutically acceptable salt thereof.
13. The compound for use according to any one of embodiments 1-12, wherein the use is intended for a patient whose level of a compound of formula (I), or a pharmaceutically acceptable salt thereof, in a blood, plasma, tissue, saliva, and/or serum sample of the patient is measured during the use.
14. The compound for use according to any one of embodiments 1-13, wherein the use is intended for a patient whose level of a compound of formula (IV), or a pharmaceutically acceptable salt thereof, in a blood, plasma, tissue, saliva, and/or serum sample of the patient is measured during the use.
15. The compound for use according to any one of embodiments 1-14, wherein the use is intended for a patient whose total level of compounds of formulas (I) and (IV), or pharmaceutically acceptable salts thereof, in a blood, plasma, tissue, saliva, and/or serum sample of the patient is measured during the use.
16. The compound for use according to any one of embodiments 1-15, wherein the use is intended for a patient whose presence and/or expression level of miR-124 in a blood and/or tissue sample of the patient is measured prior to and/or during the use.
17. The compound for use according to any one of embodiments 1-16, wherein the use is intended for a patient selected by a measured presence and/or expression level of miR-124 in a blood and/or tissue sample of the patient.
18. The compound for use according to any one of embodiments 1-17, wherein an algorithm that combines miR-124 level and the level of a cytokine or another biomarker, or levels of compounds of formulas (I) and/or (IV) as defined above or pharmaceutically acceptable salts thereof, is used to monitor severity of a disease, disorder, or condition, and/or to monitor efficacy of a treatment.
19. The compound for use according to any one of embodiments 1-18, wherein an algorithm that combines miR-124 level and the level of a cytokine or another biomarker or levels of compounds of formulas (I) and (IV) as defined above is used to select patients for a treatment.
20. An algorithm that combines miR-124 level and the level of a cytokine or another biomarker, or levels of compounds of formula (I) and (IV) as defined above to monitor severity of cancer or dysplasia, and/or to monitor efficacy of a treatment.
21. A method for treating cancer or dysplasia in a patient in need thereof comprising administering to the patient a compound of Formula **(I):** 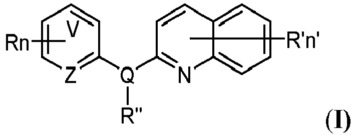 or a pharmaceutically acceptable salt thereof, wherein:
   Z is C or N;
   V is C or N; 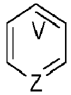 means an aromatic ring wherein V is C or N, and when V is N, V is ortho, meta or para relative to Z;
   each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R₁, -NR₁-C(=O)-R₁, -NR₁-C(=O)-NR₁R₂, -SO₂-NR₁R₂,-SO₃H, -O-SO₂-OR₃, -O-P(=O)-(OR₃)(OR₄), -O-CH₂-COOR₃, (C₁-C₃)alkyl, said alkyl being optionally mono- or di-substituted by a hydroxyl group or a group of formula (IIa): 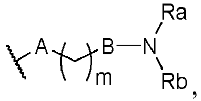 or a group of formula (IIIa): 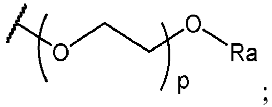
   Q is N or O, provided that R" does not exist when Q is O;
   each of R₁ and R₂ is independently hydrogen or (C₁-C₃)alkyl;
   each of R₃ and R₄ is independently hydrogen, Li+, Na⁺, K⁺, N⁺(Ra)₄ or benzyl;
   n is 1, 2 or 3;
   n' is 1, 2 or 3;
   each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy, -O-P(=O)-(OR₃)(OR₄), -CN, a group of formula (IIa): 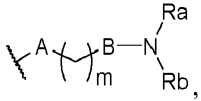 or a group of formula (IIIa): 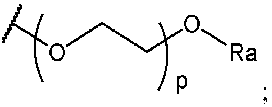
   A is a covalent bond, oxygen, or NH;
   B is a covalent bond or NH;
   m is 1, 2, 3, 4 or 5;
   p is 1, 2 or 3;
   each of Ra and Rb is independently hydrogen, (C₁-C₅)alkyl, or (C₃-C₆)cycloalkyl, or Ra and Rb form together with the nitrogen atom to which they are attached a saturated 5- or 6-membered heterocycle, said heterocycle being optionally substituted by one or more Ra, provided that when R' is a group (IIa) or (IIa), n' may be 2 or 3 only if other R' groups are different from said group (IIa) or (IIa); and
   R" is hydrogen, (C₁-C₄)alkyl, or a group of formula (IIa) as defined herein.
22. The method of embodiment 21, wherein the compound is of formula (**Ia**): 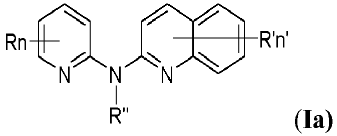 or a pharmaceutically acceptable salt thereof.
23. The method of embodiment 21, wherein the compound is of formula (**Ib**): 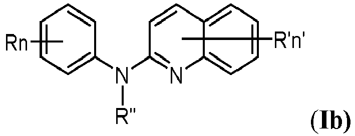 or a pharmaceutically acceptable salt thereof.
24. The method of embodiment 21, wherein the compound is of formula **(Ic):** 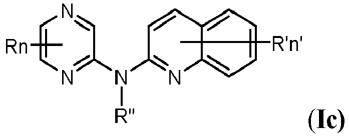 or a pharmaceutically acceptable salt thereof.
25. The method of embodiment 21, wherein the compound is of formula (**Ib'**): 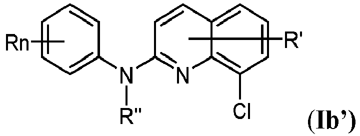 or a pharmaceutically acceptable salt thereof.
26. The method of embodiment 21, wherein the compound is of formula **(Id):** 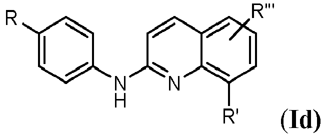 or a pharmaceutically acceptable salt thereof.
27. A method for treating cancer or dysplasia in a patient in need thereof, comprising administering to the patient a compound of formula **(IV):** 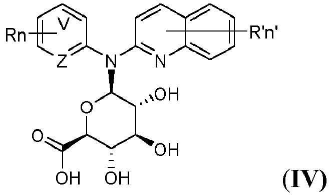 or a pharmaceutically acceptable salt thereof, wherein each of variables V, Z, R, R', n, and n' is as described in embodiment 21.
28. The method of embodiment 27, wherein the compound is of formula **(IVa):** 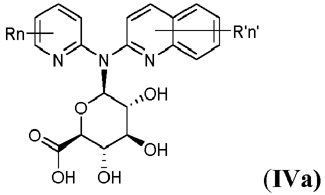 or a pharmaceutically acceptable salt thereof.
29. The method of embodiment 27, wherein the compound is of formula (**IVb**): 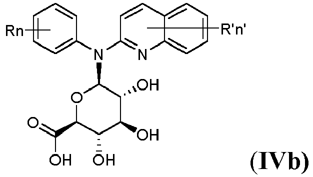 or a pharmaceutically acceptable salt thereof.
30. The method of embodiment 27, wherein the compound is of formula (**IVc**): 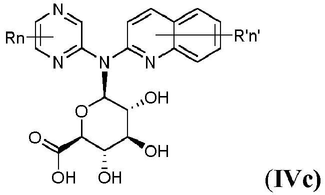 or a pharmaceutically acceptable salt thereof.
31. The method of embodiment 27, wherein the compound is of formula **(IVb'):** 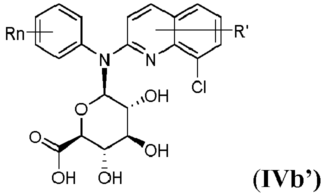 or a pharmaceutically acceptable salt thereof.
32. The method of embodiment 27, wherein the compound is of formula **(IVd):** 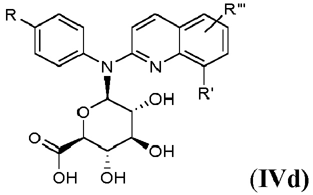 or a pharmaceutically acceptable salt thereof.
33. The method of any one of embodiments 21-32, further comprising measuring a level of a compound of formula I, or a pharmaceutically acceptable salt thereof, in a blood, plasma, tissue, saliva, and/or serum sample of the patient.
34. The method of any one of embodiments 21-33, further comprising measuring a level of a compound of formula IV, or a pharmaceutically acceptable salt thereof, in a blood, plasma, tissue, saliva, and/or serum sample of the patient.
35. The method of any one of embodiments 21-34, further comprising measuring a total level of compounds of formulas I and IV, or pharmaceutically acceptable salts thereof, in a blood, plasma, tissue, saliva, and/or serum sample of the patient.
36. The method of any one of embodiments 21-35, further comprising measuring a presence and/or expression level of miR-124 in a blood and/or tissue sample of the patient, prior to and during the course of the treatment.
37. The method of any one of embodiments 21-36, further comprising selecting a patient by a measured presence and/or expression level of miR-124 in a blood and/or tissue sample of the patient.
38. The method of any one of embodiments 21-37, further comprising using an algorithm that combines miR-124 level and the level of a cytokine or another biomarker, or levels of compounds of formulas (I) and/or (IV) as defined above or pharmaceutically acceptable salts thereof, to monitor severity of cancer or dysplasia, and/or to monitor efficacy a treatment.
39. The method of any one of embodiments 21-38, further comprising using an algorithm that combines miR-124 level and the level of a cytokine or another biomarker, or levels of compounds of formulas (I) and/or (IV) as defined above or pharmaceutically acceptable salts thereof, to select patients for treatment.
40. An algorithm that combines miR-124 level and the level of a cytokine or another biomarker, or levels of compounds of formulas (I) and/or (IV) as defined above or pharmaceutically acceptable salts thereof, to monitor severity of cancer or dysplasia, and/or to monitor efficacy of a treatment.
41. The method of any one of embodiments 21-40, further comprising measuring a level of a compound of formula (I), or a pharmaceutically acceptable salt thereof, in a blood, plasma, tissue, saliva, and/or serum sample of the patient.
42. The method of any one of embodiments 21-40, further comprising measuring a level of a compound of formula (IV), or a pharmaceutically acceptable salt thereof, in a blood, plasma, tissue, saliva, and/or serum sample of the patient.
43. The method of any one of embodiments 21-40, further comprising measuring a total level of compounds of formulas (I) and (IV), or pharmaceutically acceptable salts thereof, in a blood, plasma, tissue, saliva, and/or serum sample of the patient.

## Claims

1. **A quinoline derivative** of formula (I') 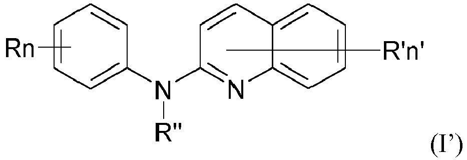 wherein:
R independently represent a halogen atom or a group chosen among a (C₁-C₃)fluoroalkyl group, a (C₁-C₃)fluoroalkoxy group, a -NR₁R₂ group, a (C₁-C₄)alkoxy group and a (C₁-C₃)alkyl group, said alkyl being optionally mono or di-substituted by a hydroxyl group,
n is 1 or 2,
n' is 1 or 2,
R₁ and R₂ are independently a hydrogen atom or a (C₁-C₃)alkyl group,
R' independently represent a halogen atom or a group chosen among a (C₁-C₃)alkyl group, a hydroxyl group, a -NR₁R₂ group, a morpholinyl or a morpholino group, a N-methylpiperazinyl group, a (C₁-C₃)fluoroalkyl group and a (C₁-C₄)alkoxy group, and can further be a group chosen among: 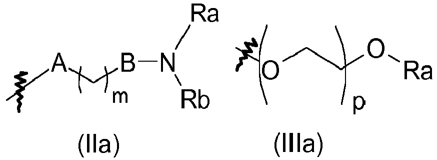
A is a covalent bond, an oxygen atom or NH,
B is a covalent bond or NH,
m is 2, 3 or 4,
p is 1, 2 or 3,
Ra and Rb independently represent a hydrogen atom, a (C₁-C₅)alkyl group or a (C₃-C₆)cycloalkyl group,
Ra and Rb can further form together with the nitrogen atom to which they are attached a saturated 5- or 6-membered heterocycle optionally containing a further heteroatom chosen among N, O and S, said heterocycle being optionally substituted by one or more Ra, provided that when R' is a group (IIa) or (IIIa), n' may be 2 only if the other R' groups is different from said group (IIa) or (IIIa), R" is a hydrogen atom or a (C₁-C₄)alkyl group,
or anyone of its metabolites or pharmaceutically acceptable salt or anyone of their enantiomers or diastereoisomers,
for use for treating or preventing cancer or dysplasia.

2. A quinoline derivative of formula (I') or anyone of its metabolites or pharmaceutically acceptable salt or anyone of their enantiomers or diastereoisomers for use according to claim 1, wherein R independently represent a methyl group, a methoxy group, a trifluoromethyl group, a halogen atom and more particularly a fluorine or chlorine atom, a trifluoromethoxy group and an amino group.

3. A quinoline derivative of formula (I') or anyone of its metabolites or pharmaceutically acceptable salt or anyone of their enantiomers or diastereoisomers for use according to claim 1 or 2, wherein R' independently represent a halogen atom and more particularly a fluorine or chlorine atom, a -NR₁R₂ group, and preferably an amino group, a hydroxyl group, a (C₁-C₃)alkyl group, preferably a methyl group, or a group 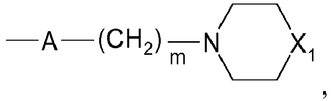 wherein A is O or NH, m is 2 or 3 and X₁ is O, CH₂ or N-CH₃, provided that when R' is such a group, n' is 1 or 2, and when n' is 2, the other R' group is different from said group.

4. A quinoline derivative of formula (I') or anyone of its metabolites or pharmaceutically acceptable salt or anyone of their enantiomers or diastereoisomers for use according to anyone of the preceding claims, wherein R' independently represent a halogen atom and more particularly a fluorine or chlorine atom, a methyl group or a group 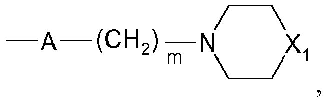 wherein A is O or NH, m is 2 or 3 and X₁ is O, CH₂ or N-CH₃, provided that when R' is such a group, n' is 1 or 2, and when n' is 2, the other R' group is different from said group.

5. A quinoline derivative of formula (I') or anyone of its metabolites or pharmaceutically acceptable salt or anyone of their enantiomers or diastereoisomers for use according to anyone of the preceding claims, wherein n is 1, n' is 1 or 2, R" is H, R is selected from a methyl group, a methoxy group, a trifluoromethyl group, a halogen atom and more particularly a fluorine or chlorine atom, a trifluoromethoxy group and an amino group, and R' represents a halogen atom and more particularly a fluorine or chlorine atom, a methyl group or a group 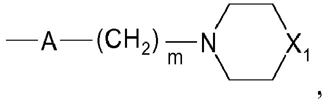 wherein A is O or NH, m is 2 or 3 and X₁ is O, CH₂ or N-CH₃, provided that when n' is 2, the other R' group is different from said group.

6. A quinoline derivative of formula (I') or anyone of its metabolites or pharmaceutically acceptable salt or anyone of their enantiomers or diastereoisomers for use according to anyone of the preceding claims, wherein n is 1, n' is 1, R" is H, R is selected from a methyl group, a methoxy group, a trifluoromethyl group, a halogen atom and more particularly a fluorine or chlorine atom and a trifluoromethoxy group, and R' represents a halogen atom and more particularly a fluorine or chlorine atom or a methyl group.

7. A quinoline derivative of formula (I") or anyone of its metabolites or pharmaceutically acceptable salt or anyone of their enantiomers or diastereoisomers for use according to anyone of claims 1-5, wherein it is defined by formula (I") 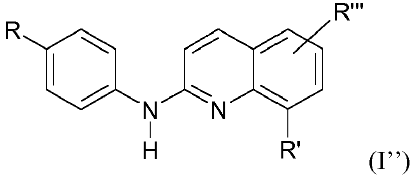 wherein
R is selected from a methyl group, a methoxy group, a trifluoromethyl group, a halogen atom and more particularly a fluorine or chlorine atom, a trifluoromethoxy group and an amino group, and
R' represents a halogen atom and more particularly a fluorine or chlorine atom or a methyl group, and
R"' represents a hydrogen atom or a group 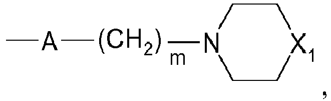 wherein A is O or NH, m is 2 or 3 and X₁ is O, CH₂ or N-CH₃, and in particular represents a hydrogen atom.

8. A quinoline derivative of formula (I') or anyone of its metabolites or pharmaceutically acceptable salt or anyone of their enantiomers or diastereoisomers for use according to anyone of the preceding claims, wherein it is 8-Chloro-N-(4-(trifluoromethoxy)phenyl)quinolin-2-amine under amorphous or crystalline form, including under the form of free base or of addition salt with pharmaceutically acceptable acids.

9. A quinoline derivative of formula (I') or anyone of its metabolites or pharmaceutically acceptable salt or anyone of their enantiomers or diastereoisomers for use according to the preceding claim, wherein 8-Chloro-N-(4-(trifluoromethoxy)phenyl)quinolin-2-amine is the polymorphic form **characterized by** signals in the X-ray powder diagram at angles 7.3; 14.6, 18.4 and 24.9 and optionally by the following additional peaks: 18.0; 24.2, 28.3 and 29.5 and even optionally by the following additional peaks: 18.6; 22.3, 23.0 and 23.5.

10. A quinoline derivative of formula (I') or anyone of its metabolites or pharmaceutically acceptable salt or anyone of their enantiomers or diastereoisomers for use according to anyone of the preceding claims, wherein it is under the form of a salt with pharmaceutically acceptable acids, in particular chosen among sulfate, hydrobromide, tartrate, citrate, trifluoroacetate, ascorbate, hydrochloride, maleate, mesylate, formate, acetate, fumarate and sulfonate, in particular alkylsufonate or arylsulfonate, and more particularly mesylate, triflate, edisylate, besylate and tosylate.

11. A quinoline derivative of formula (I') or anyone of its metabolites or pharmaceutically acceptable salt or anyone of their enantiomers or diastereoisomers for use according to anyone of the preceding claims, wherein it is a N-glucuronide metabolite.

12. A quinoline derivative of formula (I') or anyone of its metabolites or pharmaceutically acceptable salt or anyone of their enantiomers or diastereoisomers for use according to the preceding claim, wherein said N-glucuronide metabolite has the following formula **(IVa):** 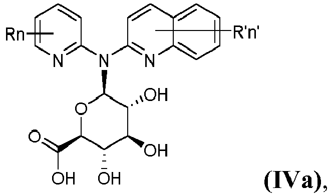 wherein R, R', n, n' are as defined in anyone of claim 1 to 7 or a pharmaceutically acceptable salt thereof.

13. A quinoline derivative of formula (I') or anyone of its metabolites or pharmaceutically acceptable salt or anyone of their enantiomers or diastereoisomers for use according to claims 11 or 12, wherein said N-glucuronide metabolite has the following formula 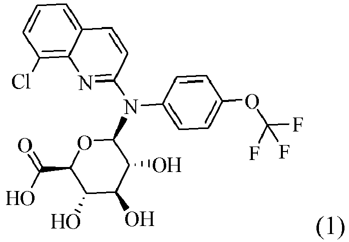 under amorphous or crystalline form.

14. A quinoline derivative of formula (I') or anyone of its metabolites or pharmaceutically acceptable salt or anyone of their enantiomers or diastereoisomers for use according to anyone of the preceding claims, wherein the cancer is selected from head and neck cancer, Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bladder Cancer, Bone Cancer, brain cancer, Brain and Spinal Cord Tumors in Adults, Brain and Spinal Cord Tumors in Children, Anaplastic astrocytomas, Breast cancer, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Cancer, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), Colorectal Cancer, colorectal adenoma, Cutaneous Squamous Cell Carcinoma, Endometrial Cancer, Epithelial Ovarian Cancer, Epithelial Ovarian Cancer associated with metastasis, oesophageal cancer, Oesophagus Squamous Cell Carcinoma, Ewing sarcoma, Ewing Family of Tumors, Lymphoblastic leukaemia (ALL), Eye Cancer, such as Ocular Melanoma and Lymphoma, Gallbladder Cancer, Gastric Cancer, gastrointestinal cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Kidney Cancer, Laryngeal and Hypopharyngeal Cancer, Leiomyosarcoma, Leukemia, Leukemia in Children, Liver Cancer, Lung Cancer, Lung Carcinoid Tumor, Lymphoma, Lymphoma of the Skin, Malignant Mesothelioma, Mantle cell lymphoma, Medulloblastoma, Melanoma Skin Cancer, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Non-Small Cell Lung Cancer, Gefitinib-resistant non-small cell lung cancer, Oral cancer, Oral Cavity and Oropharyngeal Cancer, Osteosarcoma, Pulmonary Metastatic Osteosarcoma, Ovarian Cancer, Pancreatic Cancer, thyroid carcinoma, Papillary Thyroid Carcinoma, Pediatric Spinal Ependymoma, Penile Cancer, Pituitary Tumors, Pituitary Adenoma, Proneural tumors, Prostate Cancer, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Stomach Cancer, Testicular Cancer, Thymus Cancer, Thyroid Cancer, Uterine Sarcoma, Vaginal Cancer, Vulvar Cancer, Renal cancer, Retinoblastoma, Waldenstrom Macroglobulinemia and Wilms Tumor.

15. A quinoline derivative of formula (I') or anyone of its metabolites or pharmaceutically acceptable salt or anyone of their enantiomers or diastereoisomers for use according to anyone of the preceding claims, wherein the cancer or its metastasis is selected from from a Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bone Cancer, brain cancer, Brain and Spinal Cord Tumors in Adults, Brain and Spinal Cord Tumors in Children, Anaplastic astrocytomas, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), colorectal adenoma, Cutaneous Squamous Cell Carcinoma, Endometrial Cancer, Epithelial Ovarian Cancer, Epithelial Ovarian Cancer associated with metastasis, Oesophagus Squamous Cell Carcinoma, Ewing sarcoma, Ewing Family of Tumors, Lymphoblastic leukaemia (ALL), Eye Cancer, such as Ocular Melanoma and Lymphoma, Gastric Cancer, gastrointestinal cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Laryngeal and Hypopharyngeal Cancer, Leiomyosarcoma, Leukemia, Leukemia in Children, Lung Carcinoid Tumor, Lymphoma, Lymphoma of the Skin, Malignant Mesothelioma, Mantle cell lymphoma, Medulloblastoma, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Gefitinib-resistant non-small cell lung cancer, Oral cancer, Oral Cavity and Oropharyngeal Cancer, Osteosarcoma, Pulmonary Metastatic Osteosarcoma, thyroid carcinoma, Papillary Thyroid Carcinoma, Pediatric Spinal Ependymoma, Penile Cancer, Pituitary Tumors, Pituitary Adenoma, Proneural tumors, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Testicular Cancer, Thymus Cancer, Uterine Sarcoma, Vaginal Cancer, Vulvar Cancer, Renal cancer, Retinoblastoma, Waldenstrom Macroglobulinemia and Wilms Tumor.

16. A quinoline derivative of formula (I') or anyone of its metabolites or pharmaceutically acceptable salt or anyone of their enantiomers or diastereoisomers for use according to anyone of the preceding claims, wherein the cancer or its metastasis is selected from head and neck cancer, Head and Neck Squamous Cell Carcinoma, Neck Squamous Cell Carcinoma, Malignant melanoma, stomach cancer, Breast cancer, Breast cancer in Women, Breast Cancer in Young Women, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, eye cancer, Gastric Cancer, gastrointestinal cancer, Astrocytic Glioma, Astrocytoma (grade I, II, III, or IV), colorectal cancer, colorectal adenoma, Cutaneous Squamous Cell Carcinoma, bladder cancer, bone cancer, Recurrent Breast Cancer, Hereditary Breast Cancer, HER2 positive Breast Cancer, Breast Cancer associated with lymph node metastatis, ER-alpha positive Breast Cancer, renal cancer, Cervical Intraepithelial Neoplasia, Cholangiocarcinoma, Leiomyosarcoma, Chronic Lymphocytic Leukemia (CLL), Chronic Myeloid Leukemia (CML), Chronic Myelomonocytic Leukemia (CMML), Acute Myeloid Leukemia (AML) in adults or children, Acute Lymphoblastic Leukemia,, B- or NK/T-cell lymphomas, cervical cancer, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, kidney cancer, Endometrial cancer, ovarian cancer, Epithelial Ovarian Cancer, Epithelial Ovarian Cancer associated with metastasis, Oesophageal cancer, Oesophageal Squamous Cell Carcinoma, Ewing sarcoma, Lymphoblastic leukaemia (ALL), Mantle cell lymphoma, Medulloblastoma, Lymphoma, Myelodysplastic syndrome, Meningioma, Multiple Myeloma (MM), Multiple Myeloma with Osteonecrosis of the Jaw, Nasopharyngeal Cancer, recurrent or metastatic Nasopharyngeal carcinoma, Neuroblastoma, Neuroglioma, Papillary Thyroid Carcinoma, Pediatric Spinal Ependymoma, Osteosarcoma, Pulmonary Metastatic Osteosarcoma, pancreatic cancer, thyroid carcinoma, sarcoma, pituitary tumors, Pituitary Adenoma, Proneural tumors, Squamous Cell Carcinoma of the Tongue, Mesothelioma, Retinoblastoma and prostate cancer.

17. A quinoline derivative of formula (I') or anyone of its metabolites or pharmaceutically acceptable salt or anyone of their enantiomers or diastereoisomers for use according to anyone of the preceding claims, wherein the metastasis originates from any cancer of claim 14, and is located in brain, head or neck, lung, trachea, stomach, small bowel, colon, liver, bone, peritoneum, ovary, kidney, bladder, lymph nodes, muscle or skin.

18. A quinoline derivative of formula (I') or anyone of its metabolites or pharmaceutically acceptable salt or anyone of their enantiomers or diastereoisomers for use according to anyone of the preceding claims, wherein the cancer is selected from Head and Neck cancer, Head and Neck Squamous Cell Carcinoma, Neck Squamous Cell Carcinoma, malignant melanoma, Astrocytic Glioma, Glioma, stomach cancer, Breast cancer, Cholangiocarcinoma, recurrent or metastatic Nasopharyngeal carcinoma, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, Glioblastoma, osteosarcoma, Pulmonary Metastatic Osteosarcoma, pancreatic cancer, eye cancer, gastrointestitnal cancer, colorectal cancer, colorectal adenoma, Cutaneous Squamous Cell Carcinoma, Endometrial cancer, Epithelial Ovarian Cancer, oesophageal cancer, Ewing sarcoma, gastric cancer, Hepatocellular carcinoma, HER2 positive Breast Cancer, bladder cancer, bone cancer, prostate cancer, Retinoblastoma and renal cancer.

19. A quinoline derivative of formula (I') or anyone of its metabolites or pharmaceutically acceptable salt or anyone of their enantiomers or diastereoisomers for use according to anyone of the preceding claims, wherein the cancer is selected from Anaplastic astrocytomas, Astrocytic gliomas, Bladder cancer, Breast cancer, Cholangiocarcinoma, Colorectal cancer, Colorectal adenoma, Cutaneous squamous cell carcinoma, Endometrial cancer, Epithelial ovarian cancer, Esophageal cancer, Ewing sarcoma, Gastric cancer, Gefitinib-resistant non-small cell lung cancer, Glioblastoma, Glioma, Hepatocellular carcinoma, HER2 positive breast cancer, Head and Neck Squamous Cell Carcinoma, Malignant melanoma, Nasopharyngeal carcinoma (recurrence or metastasis), Neck squamous cell carcinoma, Non-small cell lung cancer, Oral cancer, Osteosarcoma, Osteosarcoma (pulmonary metastasis), Prostate cancer and retinoblastoma.

20. A quinoline derivative of formula (I') or anyone of its metabolites or pharmaceutically acceptable salt or anyone of their enantiomers or diastereoisomers for use according to anyone of the preceding claims, wherein a presence and/or expression level of miR-124 in a blood and/or tissue sample of a patient, is measured prior to and/or during the course of the treatment, as a biomarker.

21. A quinoline derivative of formula (I') or anyone of its metabolites or pharmaceutically acceptable salt or anyone of their enantiomers or diastereoisomers for use according to anyone of the preceding claims, wherein a presence and/or expression level of miR-124 in a blood and/or tissue sample is measured to select a patient.

22. A quinoline derivative of formula (I') or anyone of its metabolites or pharmaceutically acceptable salt or anyone of their enantiomers or diastereoisomers for use according to anyone of the preceding claims, wherein a presence and/or expression level of miR-124 in a blood and/or tissue sample is measured to guide dose or monitor response to the treatment.

23. A quinoline derivative of formula (I') or anyone of its metabolites or pharmaceutically acceptable salt or anyone of their enantiomers or diastereoisomers for use according to anyone of the preceding claims, wherein miR-124 methylation is measured to guide therapy.

24. A quinoline derivative of formula (I') or anyone of its metabolites or pharmaceutically acceptable salt or anyone of their enantiomers or diastereoisomers for use according to anyone of the preceding claims, wherein an algorithm that combines miR-124 level and the level of cytokine or another biomarker, or levels of a compounds of formula (I') as defined anyone of claims 1 to 10 or anyone of its metabolites or pharmaceutically acceptable salt or anyone of their enantiomers or diasteroisomers is used to monitor severity of a cancer, and/or to monitor efficacy a treatment.

25. A quinoline derivative of formula (I') or anyone of its metabolites or pharmaceutically acceptable salt or anyone of their enantiomers or diastereoisomers for use according to anyone of claims 1 to 11 and 14 to 24, wherein the use is intended for a patient whose level of a compound of formula (I'), or a pharmaceutically acceptable salt thereof, in a blood, plasma, tissue, saliva, and/or serum sample of the patient is measured during the use.

26. A quinoline derivative of formula (I') or anyone of its metabolites or pharmaceutically acceptable salt or anyone of their enantiomers or diastereoisomers for use according to anyone of claims 1 to 11 and 14 to 24, wherein the use is intended for a patient whose level of a compound of formula (IVa), or a pharmaceutically acceptable salt thereof, in a blood, plasma, tissue, saliva, and/or serum sample of the patient is measured during the use.

27. A quinoline derivative of formula (I') or anyone of its metabolites or pharmaceutically acceptable salt or anyone of their enantiomers or diastereoisomers for use according to anyone of claims 1 to 11 and 14 to 24, wherein the use is intended for a patient whose level of a compound of formulas (I') and (IVa), or their pharmaceutically acceptable salt thereof, in a blood, plasma, tissue, saliva, and/or serum sample of the patient is measured during the use.

28. A quinoline derivative of formula (I') or anyone of its metabolites or pharmaceutically acceptable salt or anyone of their enantiomers or diastereoisomers for use according to anyone of the preceding claims, which is used in combination with another anti-tumoral agent.

29. A quinoline derivative of formula (I') or anyone of its metabolites or pharmaceutically acceptable salt or anyone of their enantiomers or diastereoisomers for use according to anyone of the preceding claims, which is used in combination with another therapy selected from chemotherapy, immunotherapy, radiotherapy, surgery, ultrasounds, monoclonal antibodies, and cancer vaccines.

30. An algorithm that combines miR-124 level and the level of a cytokine or another biomarker, or levels of a compound of formula (I') according to anyone of claims 1 to 10 or anyone of its metabolites or pharmaceutically acceptable salt or anyone of their enantiomers or diastereoisomers to monitor efficacy a treatment.
